# EUROPEAN PATENT APPLICATION

(11) **EP 2 354 789 A1**
(43) Date of publication of application: **10.08.2011**
(21) Application number: 10001267.3
(22) Date of filing: 08.02.2010
(51) Int. Cl.: G01N 33/50

(54) **Method for the identification of memory modulating compounds by assessing KIBRA expression**

(71) Applicant: Sygnis Bioscience GmbH & Co. KG, 69120 Heidelberg (DE)
(72) Inventor: Schneider, Armin, Prof. Dr., 69118 Heidelberg (DE); Spoelgen, Robert, Dr., 69120 Heidelberg (DE); Dittgen, Tanjew, Dr., 69123 Heidelberg (DE); Pitzer, Caludia, Dr., 69231 Rauenberg (DE); Plaas, Christian, 69118 Heidelberg (DE)
(74) Representative: Lahrtz, Fritz

(57) **Abstract**

Provided are cell based methods to identify memory modulating compounds based on the assessment of KIBRA expression. These methods are also suitable to uncover memory related pathways. The methods comprise the contacting of test compounds with cultured cells and the subsequent assessment of modulation of the KIBRA expression within the cells,

## Description

The present invention provides novel in vitro or cell based methods to identify memory modulating compounds based on the assessment of the expression of KIBRA gene product. These methods are also suitable to uncover memory related pathways or to test the relevance of memory modulating strategies. The provision describes assays which meet the needs in pharmaceutical research to find new treatment options for multiple neurological conditions where learning and memory is impaired. The technical specifications are described in here and exemplified below.

The protein KIBRA (for KIdney BRAin protein, also known as WWC1) has come into the focus of the neuroscience field since human genetic evidence demonstrates that a KIBRA allele is associated with memory performance and cognition in healthy subjects. It was found that carriers of the KIBRA (rs17070145) T allele performed better on multiple episodic memory tasks than those homozygous for the C allele at rs17070145 (Papassotiropoulos et al. Science 2006, 314:475; WO 2007/120955). Furthermore, stronger brain activation in key areas of learning and memory was observed in subjects carrying the C allele in comparison to T allele carriers during a memory task. The activation was measured during episodic memory task, using functional magnetic resonance imaging and suggests that non-carriers of the T allele need more activation in memory related brain regions to reach the same retrieval performance as the T allele carriers.

The association of KIBRA polymorphism with memory performance was confirmed in various studies in healthy subjects and in Alzheimer's disease patients (Schaper et al. Neurobiol Aging 2008, 29:1123; Almeida et al. J Cell Mol Med. 2008, 12:1672; Nacmias et al. Neurosci Lett. 2008, 436:145; Bates et al. Neurosci Lett. 2009, 458:140; Corneveaux et al. Neurobiol. Aging 2008, Sep. 11, e-published).

In the next decades, the aging population of the industrialized countries will suffer from an increase of different forms of memory impairments like Alzheimer's disease, aging-related cognitive decline, mild cognitive impairment, or vascular dementia. Novel treatment options are desperately needed. KIBRA may well serve as a novel pharmacological target to treat multiple forms of memory impairment, as it is linked to Alzheimer's disease and might be accessible pharmacologically (WO 2007/120955).

The role of KIBRA for neuronal plasticity is further underlined by the observation that KIBRA interacts with protein kinase C zeta (PKC zeta), a molecule crucially involved in neuronal plasticity (Büther et al. Biochem Biophys Res Commun. 2004, 317:703). It was also shown that PKC zeta can phosphorlylate KIBRA. To date, numerous scientific publications could demonstrate that PKC zeta, and also protein kinase M zeta (PKM zeta), the constitutively active form of PKC zeta, are both involved in learning and memory. A series of elegant papers fosters the influence of the kinase on memory maintenance in animals in the hippocampus (Drier et al. Nat Neurosci. 2002, 5:316; Pastalkova et al. Science 2006, 313:1141; Serrano et al. PLoS Biol. 2008, 6:2698) and in the cortex (Shema et al. Learn Mem. 2009, 16:122; Shema et al. Science 2007, 317:951). Moreover, specific inhibition of the PKM zeta activity disrupts the maintenance of LTP (Serrano et al. J Neurosci. 2005, 25:1979; WO 01/80875; WO 02/87417), a process regarded as a direct correlate of memory, whereas the activation of PKM zeta leads to increased LTP (Ling et al. Nat Neurosci. 2002, 5:295).

There is a need for reliable, efficient and non-laborious in vitro or cell based methods for the identification of new memory modulating compounds and means which can be performed in a medium to high throughput setup. These screening methods need easy to measure and robust readouts to allow multiple testing of compounds or treatments.

The embodiments characterized in the claims and herein below provide such methods based on the linkage between KIBRA and memory performance.

Accordingly, the present invention relates to in vitro or cell based methods for the identification of compounds and means capable of modulating memory performance or cognition based on the assessment of changes in KIBRA expression.

The term "expression" refers to gene expression as well as protein expression. Gene expression can be assessed e.g. by RT-PCR or micro array technologies measuring the amount of individual gene transcripts. Protein expression can be measured e.g. within an antibody based ELISA assay quantifying the amount or concentration of individual proteins. The expression level assessed according to the present invention is influenced by the synthesis rate as well as by the degradation rate or the stability of the transcript and the protein.

The term "modulation" or "modulate" means the partial or essentially complete increase or decrease.

The terms "compound" and "substance" are to be understood broadly and means, in a general manner, all the material means and substances, which can be contacted to cultured cells, e.g. but without any claim of completeness: low molecular weight compounds, peptides, polypeptides, antibodies, nucleic acids, antisense nucleic acids, siRNA, aptameres, natural or artificial transcription factors, nucleic acid constructs, vectors, viral constructs, or liposomal means.

The inventors could demonstrate that KIBRA gene expression is causatively linked to memory performance. In animal studies, rats whose KIBRA expression was elevated in the hippocampal formation, an area central to memory related pathways, maintained memory significantly better than animals of the control group (Example 1). Detailed analysis of the data revealed that KIBRA over-expression impacts the memory storage, both relevant during acquisition and retrieval in different learning paradigms, such as the Sacktor disk and the Morris water maze experiment. Accordingly, KIBRA gene expression can be enhanced in situations of increased plasticity in the hippocampus, for example by application of growth factors like G-CSF (granulocyte-colony stimulating factor) (Example 2). Increased KIBRA levels have also been seen in experiments where rats received physical rehabilitation training after cerebral ischemia, implying that increased plasticity linked to increases in KIBRA levels is not only useful for cognition and memory, but plays also a role in CNS-inherent plastic reactions to injury and disease, such as ischemic disease. This signifies that KIBRA expression levels are a correlate of increased plasticity in the hippocampus and brain. These evidences clearly suggest that enhanced KIBRA levels are linked to improved cognitive performance and general CNS plasticity.

Additionally, the inventors found that over-expression of KIBRA in cells results in both increased expression and enhanced activity of PKC zeta in cell culture experiments (Example 3). Both, increased PKC zeta levels and enhanced activity can be directly correlated with improved memory performance and vice versa (Serrano et al. J Neurosci. 2005, 25:1979; Ling et al. Nat Neurosci. 2002, 5:295; WO 01/80875; WO 02/87417).

Accordingly, one aspect of the invention is the provision of in vitro or cell culture based assays which allow to screen for KIBRA expression modulating compounds which are a highly attractive approach for the identification of memory modulators. In particular, compounds capable to enhance KIBRA expression or KIBRA activity are interesting candidates for improving cognitive performance in subjects.

In one aspect, the present invention provides methods for identification of memory and/or cognition modulating test compounds, which comprise
a) contacting said test compound with cultured cells and
b) measuring the modulation of KIBRA expression within the test cells.

To screen for compounds able to change KIBRA-expression levels one would use a cultured cell line (e.g. PC12 cells, SH-SY5Y cells, or HEK cells) or primary cells, preferentially neural cells, e.g. hippocampal cells, as the screen addresses neuronal processes.

KIBRA expression can be measured on the gene expression level (amount of mRNA) by methods known in the art such as RT-PCR (e.g. Roche LightCycler®), micro array technology (e.g. Affymetrix GeneChip®) or Northern-blotting methods, using probes complementary to the KIBRA cDNA sequence (e.g. human KIBRA (SEQ ID NO: 1), mouse KIBRA (SEQ ID NO: 3), or rat KIBRA (SEQ ID NO: 5); KIBRA cDNA sequences of further species can be identified by homology screening in cDNA libraries know to a skilled person). Such methods allow for quantitative evaluation of individual mRNA levels, and can be normalized to a reference or "housekeeping" target if necessary. Suitable primer pairs for LightCycler® based RT-PCR quantification of human KIBRA are e.g. 5'-CAC CAG AAG ACC TTA AGA GTC GA-3' (SEQ ID NO: 7) and 5'-TCA CTA TCA CTC CGA TTC AGC C-3' (SEQ ID NO: 8) and for mouse and rat KIBRA e.g. 5'-GAA GGA GCT GAA GGA GCA TTT-3' (SEQ ID NO: 9) and 5'-CCT GAA AGA CTG CAC TTC TGG-3' (SEQ ID NO: 10). A skilled person can derive further primer pairs for these and other species. Advantageously, micro array technologies allow comparisons of KIBRA levels to many other genes, and thus conclusions as to the specificity of the effects caused by a test compound.

Alternatively, alteration of KIBRA gene expression can be assessed also by using cells transfected by a reporter gene construct, wherein the reporter gene is under the control of the KIBRA promoter (as described in Example 4). Suitable report genes are e.g. without any claim to completeness: luminescence causing enzymes (such as firefly luciferase, renilla luciferase, or bacterial luciferase operon (Photorhabdus luminescens luxCDABE)), fluorescent proteins (such as green fluorescent protein (Aequorea victoria) and derivatives thereof (e.g. EYFP), or red fluorescent protein (Discosoma sp.)), substrate converting enzymes (such as beta-galactosidase, chloramphenicol acetyltransferase, beta-glucuronidase, or alkaline phosphatase), or resistance-conferring genes or else genes for growth selection.

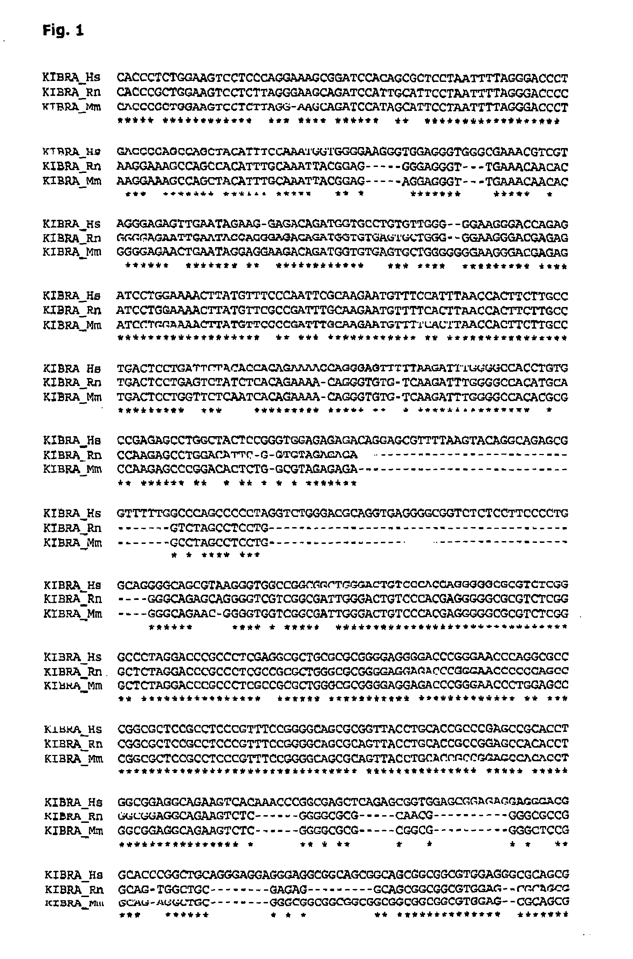

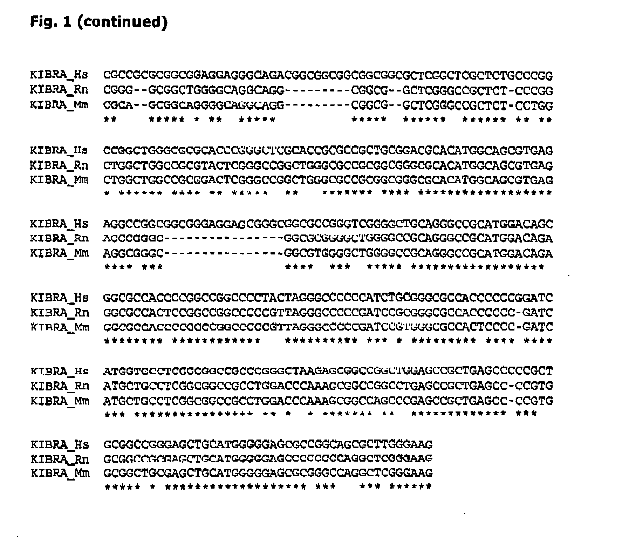

As KIBRA promoter one can use the 3 kb gene fragment upstream the KIBRA start codon (e.g. human KIBRA upstream gene fragment (SEQ ID NO: 11), mouse KIBRA upstream gene fragment (SEQ ID NO: 12), or rat KIBRA upstream gene fragment (SEQ ID NO: 13)) or any truncated fragment thereof with a length of at least 500, at least 300, or at least 100 bp, including homologue nucleic acids which are at least 90 % identical thereto, whereas the truncated fragments are preferably adjacent to the KIBRA start codon or to the KIBRA transcription start. Fig. 1 shows an alignment of the human KIBRA upstream gene fragment (SEQ ID NO: 11), the mouse KIBRA upstream gene fragment (SEQ ID NO: 12), and the rat KIBRA upstream gene fragment (SEQ ID NO: 13). The truncated fragments suitable for the use within the reporter construct according to the invention are preferably located in regions of comparable high homology of this alignment. Table 1 summarizes the localization of transcription factors binding sides predicted within the approximately 1 kb genomic upstream region of KIBRA for human, mouse and rat. These regions are particularly interesting for selection of truncated fragments suitable for the use within the reporter construct according to the invention.

One skilled in the art can isolate KIBRA promoter gene fragments of further species by homology screening using KIBRA cDNA probes and species specific genomic libraries and subsequent cloning of the genomic fragments upstream the KIBRA coding region. KIBRA-promoter containing constructs can be stably transfected in the test cell lines or transiently transfected in the test cells. Preferably, the KIBRA promoter should be species-specific for the cells used in the assay. The specificity of the promoter sequence is important in order to bind the complex set of transcription factors in the cells. Such nucleic acid constructs comprising a functional reporter gene under the control of a KIBRA promoter fragment, corresponding vectors, and host cells containing such construct are also part of the present invention. The KIBRA promoter fragment can comprise the complete 3 kb gene fragment upstream the KIBRA start codon, or one or more of the above specified truncated fragments thereof.

As a further aspect of the present invention, one can also add genomic sequences 3' to the KIBRA stop codon to such a reporter construct. Often, enhancer sites are located downstream of the coding sequences.

In addition to using the promoter, one can also assay other genomic sequences, e.g. intron 9 of the KIBRA gene. This intron will be cloned into the luciferase open reading frame, and render the luciferase ORF non-functional unless intron 9 is spliced from the pre-mRNA. As above, substance libraries can be assayed that result in enhanced luciferase activity. In the same way, all other introns in the KIBRA gene can be assayed.

**Table 1: Positions of predicted transcription factor binding sides within the approximately 1 kb 5' upstream genomic region of the human, mouse, and rat KIBRA gene. The position numbering refers to the sequences SEQ ID NO: 11, SEO ID NO: 12, and SEO ID NO: 13, respectively**

| **Transcription Factor** | **Human** | **Mouse** | **Rat** |
|---|---|---|---|
| **NFKB1** | 2880-2890; 2847-2857; 2499-2509; 2303-2313 | 2849-2859; 2563-2573 | 2882-2892; 2849-2859; 2572-2582 |
| **SPZ1** | 2904-2918; 2880-2894; 2859-2873; 2834-2848; 2795-2809; 2779-2793; 2679-2693; 2662-2676; 2633-2647; 2608-2622; 2585-2599; 2534-2548; 2513-2527; 2480-2494; 2448-2462; 2420-2434; 2399-2413; 2370-2384; 2334-2348; 2303-2317; 2254-2268; 2221-2235; 2097-2111; 2033-2047; 1947-1961 | 2905-2919; 2861-2875; 2836-2850; 2793-2807; 2768-2782; 2706-2720; 2649-2678; 2625-2639; 2598-2612; 2577-2591; 2544-2558; 2512-2526; 2484-2498; 2463-2477; 2434-2448; 2412-2426; 2243-2257; 2216-2230; 2183-2197 | 2905-2919; 2882-2896; 2849-2863; 2793-2807; 2768-2782; 2706-2720; 2689-2703; 2667-2681; 2607-2621; 2586-2600; 2553-2567; 2521-2535; 2493-2507; 2472-2486; 2420-2434; 2253-2267; 2227-2241; 2194-2208 |
| **ZNF219** | 2885-2896; 2865-2876; 2397-2408; 2095-2106 | 2866-2877; 2461-2472; 2242-2253 | 2886-2897; 2866-2877; 2470-2481; 2251-2262 |
| **MINI19_B** | 2875-2895; 2833-2853; 2807-2827; 2712-2732; 2668-2688; 2633-2653; 2589-2609; 2249-2269; 2167-2187; 2028-2048 | 2908-2928; 2877-2897; 2835-2855; 2797-2817; 2730-2771; 2698-2718; 2667-2687; 2585-2605; 2429-2449; 2386-2406; 2314-2334 | 2908-2928; 2877-2897; 2835-2855; 2803-2823; 2730-2771; 2694-2714; 2667-2687; 2594-2614; 2376-2396; 2323-2343 |
| **CBF2** | 2906-2921; 2870-2885; 2838-2853; 2818-2833; 2777-2808; 2743-2758; 2680-2695; 2653-2668; 2636-2651; 2616-2631; 2570-2585; 2532-2547; 2514-2529; 2478-2493; 2441-2456; 2421-2436; 2390-2405; 2368-2383; 2332-2347; 2288-2303; 2252-2267; 2222-2237; 2188-2203; 2159-2174; 2038-2053; 2021-2036; 1960-1975 | 2907-2922; 2872-2887; 2837-2852; 2812-2827; 2795-2810; 2766-2781; 2739-2754; 2704-2719; 2687-2702; 2670-2685; 2650-2665; 2623-2638; 2596-2611; 2578-2593; 2542-2557; 2494-2509; 2454-2469; 2432-2447; 2367-2382; 2306-2321; 2090-2105 | 2907-2922; 2870-2885; 2837-2852; 2812-2827; 2795-2810; 2766-2781; 2737-2752; 2704-2719; 2682-2697; 2646-2677; 2605-2620; 2587-2602; 2551-2566; 2534-2549; 2503-2518; 2463-2478; 2438-2453; 2376-2391; 2315-2330; 2153-2184; 2100-2115 |
| **WT1Q6** | 2911-2919; 2896-2904; 2876-2893; 2866-2874; 2852-2860; 2834-2842; 2809-2817; 2795-2803; 2779-2787; 2740-2748; 2728-2736; 2691-2699; 2679-2687; 2668-2676; 2655-2663; 2645-2653; 2633-2641; 2618-2626; 2608-2616; 2596-2604; 2585-2593; 2572-2580; 2558-2566; 2534-2542; 2519-2527; 2496-2504; 2480-2488; 2468-2476; 2446-2454; 2426-2434; 2399-2407; 2370-2378; 2357-2365; 2334-2351; 2308-2316; 2268-2276; 2254-2262; 2227-2235; 2205-2213; 2190-2198; 2178-2186; 2097-2105; 2085-2093; 2075-2083; 2059-2067; 2044-2052; 2033-2041; 2023-2031; 2001-2009; 1978-1986; 1953-1961; 1938-1946 | 2905-2913; 2886-2894; 2867-2875; 2855-2863; 2836-2844; 2805-2813; 2793-2801; 2768-2776; 2726-2734; 2706-2714; 2696-2704; 2675-2683; 2649-2666; 2616-2633; 2594-2602; 2583-2591; 2560-2568; 2550-2558; 2539-2547; 2510-2518; 2490-2498; 2463-2471; 2432-2449; 2422-2430; 2398-2406; 2378-2386; 2366-2374; 2351-2359; 2325-2333; 2281-2289; 2243-2251; 2230-2238; 2216-2224; 2201-2209; 2183-2191; 2091-2099 | 2905-2913; 2878-2895; 2867-2875; 2855-2863; 2836-2844; 2805-2813; 2793-2801; 2768-2776; 2726-2734; 2706-2714; 2687-2704; 2667-2675; 2637-2645; 2627-2635; 2603-2611; 2592-2600; 2569-2577; 2559-2567; 2548-2556; 2538-2546; 2519-2527; 2499-2507; 2472-2480; 2441-2458; 2430-2438; 2406-2414; 2387-2395; 2375-2383; 2360-2368; 2334-2342; 2290-2298; 2254-2262; 2241-2249; 2227-2235; 2212-2220; 2194-2202; 2157-2165; 2101-2109 |
| **PAX5** | 2825-2852; 2755-2782; 2561-2588 | 2813-2840; 2773-2800; 2488-2515 | 2813-2840; 2773-2800; 2658-2685; 2628-2655; 2497-2524; 2429-2456 |
| **AP2_Q6** | 2911-2922; 2885-2896; 2871-2882; 2843-2854; 2814-2825; 2800-2811; 2781-2792; 2730-2741; 2716-2727; 2667-2678; 2598-2621; 2556-2567; 2492-2503; 2465-2476; 2448-2459; 2428-2439; 2397-2408; 2380-2391; 2349-2360; 2329-2340; 2308-2319; 2249-2260; 2218-2229; 2039-2050; 1995-2006; 1955-1966; 1940-1951 | 2920-2931; 2873-2884; 2845-2856; 2816-2827; 2802-2813; 2767-2778; 2746-2757; 2692-2703; 2644-2655; 2613-2636; 2556-2567; 2529-2540; 2488-2511; 2461-2472; 2444-2455; 2419-2430; 2387-2398; 2363-2374; 2239-2250; 2198-2209; 2093-2104 | 2920-2931; 2887-2898; 2873-2884; 2856-2867; 2816-2827; 2802-2813; 2767-2778; 2746-2757; 2728-2739; 2684-2695; 2622-2645; 2565-2576; 2538-2549; 2497-2520; 2470-2481; 2453-2464; 2427-2438; 2372-2383; 2251-2262; 2209-2220; 2188-2199; 2158-2169; 2103-2114 |
| **MA0079** | 2883-2892; 2864-2873; 2844-2853; 2820-2829; 2796-2805; 2776-2785; 2764-2773; 2743-2752; 2713-2722; 2689-2708; 2669-2678; 2653-2662; 2634-2643; 2615-2624; 2559-2568; 2523-2532; 2499-2508; 2481-2490; 2448-2457; 2425-2444; 2403-2412; 2381-2390; 2354-2363; 2335-2344; 2304-2313; 2255-2264; 2205-2214; 2059-2068; 2041-2050; 2024-2033 | 2909-2918; 2898-2907; 2846-2855; 2822-2831; 2798-2817; 2782-2791; 2769-2778; 2756-2765; 2735-2744; 2693-2722; 2662-2671; 2650-2659; 2626-2635; 2581-2590; 2563-2572; 2545-2554; 2513-2522; 2489-2498; 2467-2476; 2408-2417; 2351-2360; 2243-2252; 2184-2193; 2090-2099 | 2909-2918; 2898-2907; 2885-2894; 2848-2857; 2822-2831; 2798-2817; 2782-2791; 2769-2778; 2756-2765; 2731-2740; 2703-2722; 2689-2698; 2668-2677; 2646-2655; 2628-2637; 2590-2599; 2572-2581; 2554-2563; 2522-2531; 2498-2507; 2476-2485; 2433-2442; 2360-2369; 2190-2199; 2169-2178; 2100-2109 |
| **KROX6** | 2883-2896; 2862-2875; 2781-2794; 2667-2680; 2640-2653; 2610-2623; 2426-2439; 2397-2410; 2306-2319; 2095-2108; 2039-2052 | 2864-2877; 2792-2805; 2767-2780; 2644-2657; 2490-2503; 2461-2474; 2242-2255; 2091-2104 | 2885-2898; 2864-2877; 2792-2805; 2767-2780; 2683-2696; 2499-2512; 2470-2483; 2251-2264; 2193-2206; 2101-2114 |
| **CACD** | 2884-2891; 2839-2846; 2797-2804; 2788-2795; 2727-2734; 2647-2654; 2610-2617; 2595-2602; 2518-2525; 2485-2492; 2450-2457; 2425-2432; 2404-2411; 2307-2314; 2256-2263; 2207-2214; 2100-2107; 2061-2068; 2035-2042 | 2885-2892; 2853-2860; 2841-2848; 2799-2806; 2762-2769; 2651-2658; 2620-2627; 2549-2556; 2514-2521; 2489-2496; 2468-2475; 2353-2360; 2247-2254 | 2886-2893; 2853-2860; 2799-2806; 2762-2769; 2725-2732; 2641-2648; 2629-2636; 2558-2565; 2523-2530; 2498-2505; 2477-2484; 2362-2369; 2256-2263; 2196-2203 |
| **LRF2** | 2883-2891; 2862-2870; 2850-2858; 2839-2847; 2812-2820; 2499-2507; 2453-2470; 2401-2409; 2359-2367; 2347-2355; 2334-2342; 2307-2315; 2224-2232; 2101-2109; 2029-2037; 2001-2009 | 2917-2925; 2885-2893; 2864-2872; 2852-2860; 2841-2849; 2814-2822; 2698-2706; 2631-2639; 2619-2627; 2563-2571; 2513-2521; 2465-2473; 2435-2443; 2425-2433; 2369-2377; 2248-2256; 2204-2212 | 2917-2925; 2885-2893; 2864-2872; 2852-2860; 2814-2822; 2690-2707; 2640-2648; 2628-2636; 2572-2580; 2535-2543; 2522-2530; 2474-2482; 2443-2451; 2433-2441; 2378-2386; 2257-2265; 2215-2223; 2194-2202; 2154-2162 |

Recently, it has become obvious that the level of gene expression regulation on the mRNA level is much higher and more complex than previously assumed. mRNA stability can be affected by proteins binding to the 3'UTR for example. Therefore, the present invention also provides methods for identification of memory and/or cognition enhancing test compounds, which comprise the constitutive expression of full length KIBRA mRNA in the culture cells, contacting said cells with the test compound, and measuring the amount of KIBRA transcript in comparison to control cell (Example 5). Suitable expression constructs comprise the full length KIBRA cDNA (e.g. human KIBRA (SEQ ID NO: 1), mouse KIBRA (SEQ ID NO: 3), or rat KIBRA (SEQ ID NO: 5) under the control of a eukaryotic promoter such as CMV or SV40. Said KIBRA cDNA comprises the coding region and at least 50 bp of the corresponding 5'UTR and/or at least 100 bp of the corresponding 3'UTR. The amount of the KIBRA transcript can be measured e.g. with RT-PCR based methods such as LightCycler®. In order to examine this stability effect isolated from potential influences on the KIBRA promoter, one can use for this assay cell without exogenous KIBRA expression, or cells derived from a species whose KIBRA sequences distinguishes sufficiently enough from the one use in the expression construct. and thereby, allow for separate detection.

Alternatively, alteration of KIBRA expression can be assessed on the protein level (amount of protein) by methods known in the art such as ELISA, Western-blot, or immunoprecipitation using specific anti-KIBRA antibody. Detection of KIBRA protein level can be performed by using KIBRA specific antibodies. Such antibodies can be generated form mouse hybridoma cells, which have been generated e.g. by the immunization of mice using KIBRA peptides, in order to receive specific monoclonal antibodies. Alternatively, polyclonal antibodies isolated from the serum of animals immunized with KIBRA protein or peptides thereof can be used accordingly. Suitable KIBRA peptides can be derived form the KIBRA protein sequences (e.g. human KIBRA protein (SEQ ID NO: 2), mouse KIBRA protein (SEQ ID NO: 4), or rat KIBRA protein (SEQ ID NO: 6)).

Furthermore, the present invention also provides methods for identification of memory and/or cognition enhancing test compounds, which comprise the constitutive expression of KIBRA protein or a KIBRA fusion protein in the cultured cells, contacting said cells with the test compound, and measuring the amount of KIBRA protein or KIBRA fusion protein in comparison to control cell (Example 6). Compounds that influence the stability of KIBRA protein can be identified within this assay method. The amount of KIBRA protein and KIBRA fusion protein can be quantified by antibody based methods such as ELISA using specific anti-KIBRA antibodies. In KIBRA fusion proteins the KIBRA protein is linked to a tag peptide such as myc-tag or FLAG-tag, which allows detection with commercially available antibodies, or to a reporter protein such as luciferase which allows direct quantification of the fusion protein.

Also, KIBRA expression can be monitored utilizing transgenic cells whereas the KIBRA gene is partly replaced by a reporter construct, such as the luciferase cDNA resulting in a chimaeric protein under the control of the original KIBRA promoter of the cells. One such construct could be a fusion of the full-length luciferase cDNA subsequently to the 3'-end of the KIBRA coding region.

A test compound is considered as screening hit according to the invention if KIBRA expression is modulated in the cultured cells by at least 20 %, compared to control cells and/or if KIBRA expression is modulated statistically significantly in cultured cells compared to control cells. Whether a portion is statistically significant can be determined without further ado by the person skilled in the art using various well known statistic evaluation tools, e.g., determination of confidence intervals, p-value determination, Student's t-test, Mann-Whitney test etc. Preferred confidence intervals are at least 95 %. The p-values are, preferably, 0.05.

Test compounds for this assay method can be for example polypeptides, proteins (including chimeric proteins), antibodies, nucleic acids (e.g. siRNA), gene therapy approaches (virus) or small molecules. Preferable for a high throughput appoach are small molecules, with a molecular weight of less than 2,000 g. Compounds are generally diluted in salt buffer of physiological concentration (e.g. PBS), hydrophobic compounds, like fat-soluble vitamins, can be diluted in suitable solvents, e.g. 20 % Solutol HS (BASF, Ludwigshafen, Germany), for efficient application to the cell culture.

In the view of the above, a method according to the invention that identifies compounds enhancing KIBRA expression or activity is a promising approach for treating and/or preventing cognition impairments, including non-pathological forms like "age related memory loss" (ARML). Cognitively impaired people have difficulty with one or more of the basic functions of their brain, such as perception, memory, concentration and reasoning skills. Common causes of cognitive impairment include Alzheimer's disease and related dementias, stroke, Parkinson's disease, brain injury, brain tumor or HIV-associated dementia.

Accordingly, one aspect of the present invention is the use of compounds, identified by a method of the invention as being capable to enhance the KIBRA expression, for the manufacturing of a pharmaceutical preparation for enhancing memory and cognitive ability, and thereby, treating disease such as Alzheimer's disease, dementia, or "mild cognitive impairment" (MCI) and also non-pathological ARML.

Also, one aspect of the present invention is a method for improving memory or cognitive ability in a subject, the method comprising administering to said subject in need thereof, a therapeutically effective amount of a compound, identified by a method of the invention as being capable to enhance the KIBRA expression, particularly, whereas said subject suffer from Alzheimer's disease, dementia, MIC, or non-pathological ARML.

### Brief description of the figures

Fig.1: Alignment of the approximately 1 kb genomic sequence upstream of the coding region start of the human, mouse and rat KIBRA promoter region (KIBRA_hs: human genomic sequence, bases 1940 to 3000 of SEQ ID NO: 11; KIBRA_mm: mouse genomic sequence, bases 2093 to 3000 of SEQ ID NO: 12; KIBRA_rn: rat genomic sequence, bases 2103 to 3000 of SEQ ID NO: 13). Asterisks mark positions of the alignment with bases identical over all three species.

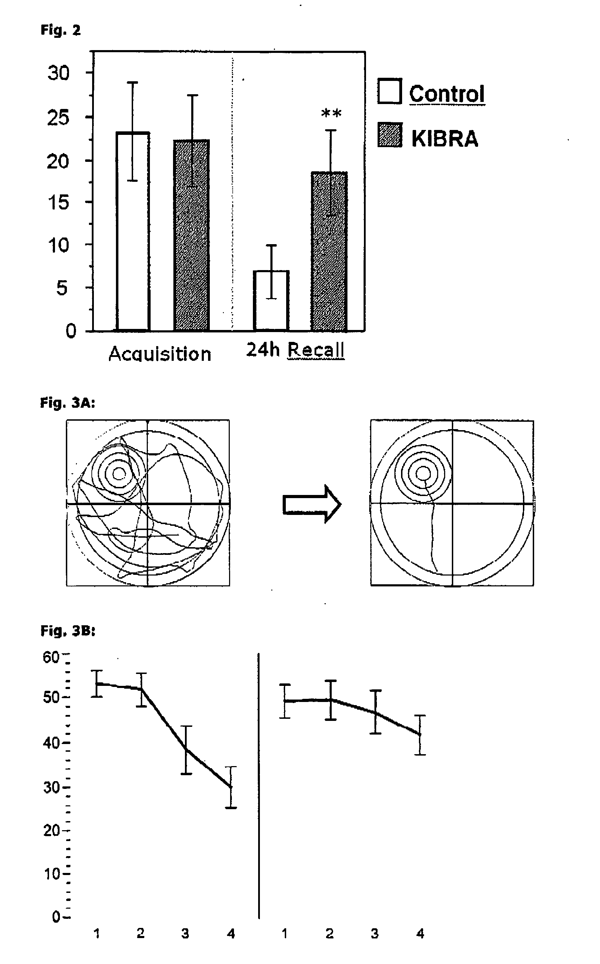

Fig. 2: Sacktor disk results for KIBRA over-expressing (KIBRA) and control (CONTROL) animal; bars show the time to enter the shock sector in seconds for the rats of the two groups for the acquisition (9^{th} trial) and for the recall trial after 24 h. KIBRA over-expressing animals perform significantly better in the recall trial by avoiding the shock sector for a longer time. Error bars represent the SEM.
Fig. 3 A: Depiction of traces of the test rat obtained during water maze learning, at the beginning of the learning trials and at the end. Whereas in the initial trial, the rat searches for the hidden platform throughout the complete basis, at the end of the trials, the rat knows where to find the hidden platform.
Fig. 3 B: Comparison of learning speed (mean of the time (sec) to platform for the test rats) in the first 4 learning runs between KIBRA over-expressing rats (left panel) and control rats (right panel). Error bars represent the SEM. After initial learning trials, KIBRA over-expressors need less time to find the hidden platform compared to the control animals.

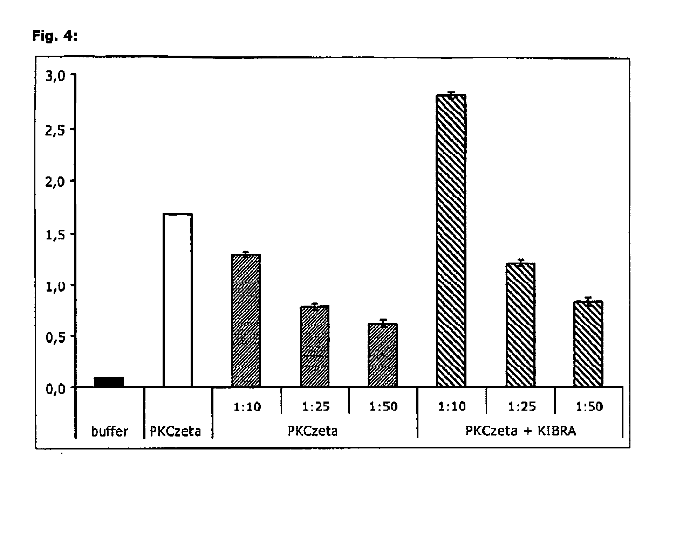

Fig. 4: Induction of PKC zeta activity by KIBRA. The hatched bars show the PKC zeta kinase activity in the absence (PKCzeta) and in the presence (PKCzeta + KIBRA) of KIBRA over-expression, whereas the PKC zeta activity was measured, after affinity purification and normalization to the PKC zeta protein amount, in various dilutions (1:10, 1:25 und 1:50). The black bar (buffer) is the base line control of the PKC zeta kinase assay without addition of any PKC zeta protein. The white bar (PKCzeta) shows the positive control of the PKC zeta kinase assay in the presence of 5 ng PKC zeta protein.

### Examples

### Example 1:

### Over-expression of KIBRA in the hippocampus of rats.

KIBRA was over-expressed in the hippocampal region of rats. The over-expression was mediated by Adeno-associated virus 2 (AAV2) KIBRA injection via stereotactic surgery in the hippocampal region of both brain hemispheres of adult male rats (3 month of age). After three weeks animals either treated with AAV KIBRA or an AAV expressing GFP (n = 20, each group), were subjected to behavioural tests.

KIBRA over-expressors showed a strong improvement in memory performance in a specific form of active place avoidance, herein called "Sacktor disk". The Sacktor disk provides the experimental advantages of rapid hippocampus-dependent acquisition and persistent hippocampus-dependent recall (Pastalkova et al. Science 2006, 313:1141). The apparatus consists of a slowly rotating platform disk (1 rpm), open to the room environment. The rotation of the platform carries the animals into a 60° shock sector, which can be energized, forcing the animals to actively avoid the shock sector. A video camera connected with a tracking system is installed above the maze to record the movement of the animals.

For the analysis animals are first subjected to a habituation trial, in which the animals are exposed to the apparatus for 10 min without shock. After the habituation animals continue with successive training trials, in which the animals receive an electric shock every time the animals run into the shock zone (0.6 mA for 0.5 s every 1.5 s). Training consists of eight 10 min training trials, separated by 10 min rest intervals in their home cage.

Retention of long term memory is tested after 24 h in a single probe trial for 10 min, in the absence of shock. The performance of the animals is determined by the time the animal first entered the shock zone, total visits of the shock zone and number of shocks (time in shock zone).

KIBRA AAV treated rats performed significantly better than the control group, as the animals avoided the shock zone for a longer time (Fig. 2). In line with the time KIBRA over-expressing animals to avoid the shock zone, KIBRA over-expressors also showed significantly less visits in the shock zone.

Another way to measure cognition and memory is the Morris water maze test. The Morris water maze has been extensively used to analyze spatial learning and memory (Devan et al. Neurobiol Learn Mem. 1996, 66:305). The experimental procedure involves training rodents to find a hidden platform in a large, circular pool of water.

A water maze (diameter 180 cm) was filled with an opaque mixture of black, watersoluble cola-paint and was heated to 25 °C for trials. A collapsible Plexiglas platform is used (12 cm x 12 cm surface) extended from the floor of the tank to 1.5 cm below the surface of the water. The tank is exposed to the room environment with different visual cues are positioned at the north, east, south and west of the tank, respectively. A video camera connected with a tracking system is installed above the maze to record the movement of the animals.

In the training trials, the platform is placed at a fixed position, lowered to 1.5 cm below the surface of the water, that the animals were unable to see the platform. On each trial, the rat was placed in the pool along the perimeter of the tank facing the tank wall. The four entry points, North, East, South, and West, were randomized across trials, but the same sequence of start points was used for each rat. The animals were allowed to reach the platform in 60 sec on each trial. Trials ended when the rat located the escape platform, or when 60 sec had elapsed, at which time the rat was guided to the platform. In either case, the rat was allowed to remain on the platform for 30 sec before being returned to a holding cage for a 30-sec inter-trial interval. Animals are tested for 4 trials each day for 4 d.

The time animals needed to reach the platform during each training trial was measured. After the training animals underwent a probe trial on the 5^{th} day in which the platform was completely removed. The animals are placed to the opposite quadrant of the platform quadrant and allowed to search for the platform for 120 sec. The time the animals spent in the quadrant and the distance over time to the place where the platform was originally located was recorded, as well as the distance animals covert in the maze. Analysis of the experiment showed that animals injected with the AAV-Virus leading to KIBRA over-expression performed significantly better in learning the location of the hidden platform than animals injected with the control AAV-Virus. Using multiple linear regression analysis with the outcome variable TIME TO PLATFORM and with the factors VIRUS TREATMENT, TRIAL NUMBER, STARTING POSITION, and VIRUS TREATMENT* TRIAL NUMBER both VIRUS TREATMENT itself and the interaction term were significantly different (VIRUS TREATMENT: p=0.0045; VIRUS TREATMENT TRIAL NUMBER: p= 0.0152). Fig. 3A shows an example of traces obtained during water maze learning, Fig. 3B shows a comparison of learning speed in the first 4 learning runs, with a clear advantage for KIBRA over-expressors.

Thus, in two different experimental learning paradigms KIBRA over-expression resulted in a significant improvement in learning and memory performance.

### Example 2

### G-CSF enhances KIBRA expression.

The neuroprotective and neuroregenerative molecule Granulocyte-Colony Stimulating Factor (G-CSF) has effects on cognition. Synergetic effects of granulocyte-colony stimulating factor and cognitive training on spatial learning and survival of newborn hippocampal neurons have also been shown (Diederich et al. PLoS One 2009, 4:5303). The role of G-CSF in the healthy brain has been examined by the characterization of G-CSF-deficient mice (Diederich et al. J Neurosci 2009, 29:11572). In the present example, we tested the pro-cognitive G-CSF in anxiogenic and learning and memory paradigms in the mouse. After AAV-mediated delivery of G-CSF into the hippocampus of mice, no anxiogenic effects were detected, whereas spatial reference and working memory was improved in the G-CSG-treated group.

AAV constructs expressing the human G-CSF protein were stereotactically injected bilaterally into the hippocampus of 10 C57BL\6 mice at four sites (AP -1.3 mm / L ±1.8 mm / DV -2 mm and AP -0.7 mm / L ± 2.4 mm / DV -2 mm). For controls, 10 mice were injected with the empty vector.

In the T-maze, the non-matching-to-place paradigm (NMTP) was tested for spatial working memory. In the T-shaped plastic apparatus, mice were trained in 10 trials per day, consisting of two runs each. In the first run, mice were allowed to enter only one arm of the T-maze containing sweetened milk. In the second run, both arms were available for choice, with the food reward placed in the opposite arm. In the paddling pool for testing spatial reference memory, mice were placed in the circular apparatus of 115 cm in diameter with 12 exit holes, of which 11 holes were blocked. In 5 trials per day, the animals were trained to find the unblocked exit always located at 12 o'clock. After 7 days, the exit position was rotated to 4 o'clock. The time until the new exit position was found and the number of errors was recorded.

Analysis of the results showed that G-CSF-over-expressing animals perform superior to AAV-control-injected animals in the T-maze test described above.

To analyze what molecular mechanisms contributed to enhanced cognition induced by G-CSF, we conducted a gene expression study of the hippocampus tissue. Gene expression was analyzed after Laser-microdissection of the hippocampus, amplification of mRNA, and hybridization to micro arrays (GeneChip®, Affymetrix, Santa Clara, CA, USA). Data were analyzed using Genesifter (Geospiza Inc., Seattle, WA, USA). The KIBRA mRNA was found significantly induced by a factor of 3.09-fold (Affymetrix ID 1427261_at; HECT C2 and WW domain containing E3 ubiquitin protein ligase 1).

This example demonstrates that KIBRA can be induced by pharmacological means, and that an increase in KIBRA correlates to improved cognitive capabilities.

### Example 3

### PKC zeta activity is regulated by KIBRA

To investigate the impact of KIBRA on PKC zeta activity itself, PKC zeta activity were determined in an enzyme assay. The kinase activity in the assay is detected by the phosphorylation of a substrate peptide by an antibody reaction according to manufactures instruction (HTScan PKC zeta kinase assay kit, Cell Signaling Technology Inc., Danvers, MA, USA). The antibody is detected by a lanthanide-labeled secondary antibody, which can be measured in a fluorescent plate reader.

In order to analyze PKC zeta activity with and without KIBRA, COS cells over-expressing PKC zeta alone or in combination with KIBRA were lysed and PKC zeta was pulled down with respective PKC zeta binding beads. To normalize for equal pull down of PKC zeta, aliquots of the pull downs were subjected to western blot analysis and the resulting PKC zeta signal in the blot were used to normalize for comparable amounts in the activity assay.

Remarkably, the amount of PKC zeta that was co-immunoprecipitated was similar between cells only over-expressing PKC zeta to cells over-expressing PKC zeta and KIBRA.

Previous studies have shown that PKC zeta co-immunoprecipitates with KIBRA, and immunoreactive bands for KIBRA in the western blot analysis confirmed that PKC zeta pulldowns include KIBRA protein from cells also over-expressing KIBRA.

When pulldowns from cells over-expressing PKC zeta were compared to cells over-expressing both PKC zeta and KIBRA for the phosphorylation of substrate peptide in the activity assay, a strong increase of PKC zeta activity by 50 % was observed. Verification of specificity of the assay was determined by pulldown of a truncated inactive form of PKC zeta. Analysis of the kinase activity of the truncated form confirmed that the phosphorylation of the peptide was only mediated by PKC zeta and no other kinase attached to the beads.

Analysis of different amounts of pulldowns form cells with and without KIBRA finally confirmed a significant and strong increase of PKC zeta activity when KIBRA is present.

### Example 4

In vitro analysis of KIBRA Expression in the presence of a test compound using genomic sequences

One approach to alter expression of a particular gene is to target regulatory sequences directly, or indirectly (e.g. via transcription factors, splicing factors, or enhancer factors). For this, a reporter gene (such as luciferase) can be cloned after the human KIBRA promotor (e.g. the first 3000 bp 5' to the start codon). This construct will be transfected into cells, ideally SHSY-5Y cells. These cells will be grown in 96- or 384-well plates at densities of 5000 - 30000 cells. Substances, will be applied to the cells at concentrations of 1 or 10 µM, in several replicates per substance. Luciferase amount present per well can be determined by luciferase plate readers. Substances that result in statistically significant elevation of luciferase activity beyond 20 % are considered as "hits".

### Example 5

In vitro analysis of KIBRA Expression in the presence of a test compound using 5' and 3' mRNA UTR sequences

For a screening approach, the full mRNA of human KIBRA will be cloned in a CMV vector, purified, and transfected into SHSY-5Y cells. Ideally, a stable transfectant will be established. Cells will then be grown on a 96 well plate at a density of 10.000 - 20.000 cells per well. Alternatively, cells can be grown on 384-well plates at a density of 5.000 - 10.000 cells per well. Small-molecule substances will be given to the wells in a concentration of 1 and 10 µM in 8 replicates per concentration. After 4 days in culture, RNA will be extracted from the cells, and analyzed by quantitative RT-PCR in comparison to an endogenous mRNA (cyclophilin). After statistical analysis, substances will be identified that produced a significant elevation (or depression) of KIBRA (WWC1) mRNA levels. A meaningful factor for up- or down-regulation is 20 % and above, at a p value of < 0.05. These substances ("hits") will be further analyzed by doing a careful concentration curve to determine EC50, nad wit Ibe cehcekd for unspecific or unwanted effects such as cellular toxicity, apoptosis, downregulation or induction of other genes. The last point will be ensured by subjecting total mRNA extracted from cells harbouring the reporter plasmid (or having stably integrated the reporter plasmid into their genomes) after exposure to said substance to analysis by DNA-arrays, such as provided by the company Affymetrix. Statistical analysis of hybridization signals will then allow to determine relative specificity of the hit in question.

In an alternative screening approach, a reporter construct can be supplied that replaces the KIBRA open reading frame (ORF) with the coding sequence for a reporter protein such as luciferase, GFP, secreted luciferase, beta-galactosidase, or others. This construct can be transfected as described above, and the screening method conducted as described with the exception that the read-out will not be based on quantitative PCR, but on a reporter assay. Most favorably, this is a luciferase-based assay using plate readers. Results are treated as above, and substances further examined for specificity and toxicity. This approach will only identify substances that target the 5' or 3' UTR, while the first approach also allows to identify substances that target the whole mRNA sequence.

In a further alternative screening approach one relies on the endogenous gene expression. Such an approach can be performed exactly as described above but without transfecting the SHSY-5Y cells. Alternative to using SHSY-5Y cells, primary neurons can be employed, NG108 cells, NSC34 cells, PC12 cells, and other neural cells and cell lines known to the expert.

In these screening approaches, small molecule substances can be replaced by siRNA libraries, by peptide libraries, or by gene therapeutic viral constructs.

### Example 6

In vitro analysis of KIBRA Expression in the presence of a test compound targeting the stability of the KIBRA protein

For a screening approach identifying compounds which influence the stability of the KIBRA protein, constructs can be used that either express KIBRA, or a fusion protein harbouring the full KIBRA sequence, and a reporter protein, such as luciferase. If only the KIBRA protein is used, the assay will be an ELISA. SHSY-5Y cells will be transfected with a plasmid harbouring an expression cassette for KIBRA. Cells will then be plated onto 96- or 384-well plates. Substances at concentrations of 1 µM or 10 µM will be applied to the cells in replicates. An ELISA using an antibody against human KIBRA will be performed. Read-outs will be statistically analyzed, and hits identified.

Alternatively, the fusion part of the protein may be detected. If this is a small peptide tag, such as the FLAG, or myc epitope, an ELISA can be utilized detecting that unique tag.

If a reporter such as luciferase is to be detected, a luciferase assay using a plate reader will be employed.

## Claims

1. A method for identifying memory and/or cognition modulating substances, which comprises
a. contacting said substance with cultured cells and
b. assessing the modulation of KIBRA expression within the cells.

2. The method of claim 1, wherein the modulation of KIBRA expression on transcript level is assessed.

3. The method of claim 1, wherein the modulation of KIBRA expression on protein level is assessed.

4. The method of claim 2, wherein the endogenous KIBRA expression of the cells is quantified, by specific nucleic acid quantification methods, such as quantitative RT-PCR methods, by micro array methods, or by Northern blot methods.

5. The method of claim 2, wherein the cultured cells are transfected with an expression construct previous to the contacting of the cells with the test substance, whereas the expression construct comprises a reporter gene under the control of a KIBRA promoter.

6. The method of claim 5, wherein the KIBRA promoter is the 3 kb genomic region upstream of the KIBRA coding sequence or any truncated fragment with a length of at least 100 bp or a combination of tow or more of these truncated fragments.

7. A expression vector comprising a reporter gene under the control of a KIBRA promoter.

8. The expression vector of claim 7, wherein the KIBRA promoter is the 3 kb genomic region upstream of the KIBRA coding sequence, or any truncated fragment with a length of at least 100 bp, or homologous thereof having at least 90 % identity to said truncated fragments, or a combination of two or more of these truncated fragments.

9. A host cell containing the expression vector of claim 7 or 8.

10. The method of claim 2, wherein the cultured cells are transfected with an KIBRA expression coonstruct previous to the contacting of the cells with the test substance, whereas the expression construct comprises a KIBRA full length cDNA, including 3' and/or 5' untranslated regions (UTR), under the control of a exogenous promoter, and whereas the quantification of the expression level of said full length cDNA reflects the alteration of its stability due to the contacting of the cells with the test substance.

11. The method of claim 3, wherein the level of endogenous KIBRA protein is quantified using anti-KIBRA antibodies within quantitative protein detection methods such as ELISA methods, Western blot methods, or immunoprccipitation methods.

12. The method of claim 3, wherein the cultured cells are transfected with a KIBRA or KIBRA fusion protein expression construct previous to the contacting of the cells with the test substance, whereas the quantification of the expression level of said KIBRA protein or KIBRA fusion protein reflects the alteration of its stability due to the contacting of the cells with the test substance.

13. The method of claim 12, wherein the KIBRA fusion protein comprises a tag-peptide or a reporter gene polypeptide linked to the N- or the C-terminus of the KIBRA polypeptide, optionally with a linker sequence between them.

14. The method of claim 13, wherein the tag-peptide is selected from a group consisting of FLAG-peptide and myc-peptide.

15. The method of any of the claims 5, 6, or 13, the vector of claim 7 or 8, or the host cell of claim 9, wherein the reporter gene is selected from the group consisting of a luciferase, a fluorescent protein, beta-galactosidase, chloramphenicol acetyltransferase, beta-glucuronidase, alkaline phosphatase, a resistance-conferring gene, and a gene for growth selection.


SEQUENCE LISTING

<110>  SYGNIS Bioscience GmbH & CO KG

<120>  Method for the identification of memory modulating compounds by
       assessing KIBRA expression

<130>  ZNS45A

<160>  13

<170>  PatentIn version 3.3

<210>  1
<211>  4160
<212>  DNA
<213>  Homo sapiens


<220>
<221>  CDS
<222>  (67)..(3426)

<400>  1
ctggagccgc tgagcccccg ctgcggccgg gagctgcatg ggggagcgcc ggcagcgctt      60

gggaag atg ccc cgg ccg gag ctg ccc ctg ccg gag ggc tgg gag gag      108
       Met Pro Arg Pro Glu Leu Pro Leu Pro Glu Gly Trp Glu Glu
       1               5                   10

gcg cgc gac ttc gac ggc aag gtc tac tac ata gac cac acg aac cgc      156
Ala Arg Asp Phe Asp Gly Lys Val Tyr Tyr Ile Asp His Thr Asn Arg
15                  20                  25                  30

acc acc agc tgg atc gac ccg cgg gac agg tac acc aaa ccg ctc acc      204
Thr Thr Ser Trp Ile Asp Pro Arg Asp Arg Tyr Thr Lys Pro Leu Thr
                35                  40                  45

ttt gct gac tgc att agt gat gag ttg ccg cta gga tgg gaa gag gca      252
Phe Ala Asp Cys Ile Ser Asp Glu Leu Pro Leu Gly Trp Glu Glu Ala
            50                  55                  60

tat gac cca cag gtt gga gat tac ttc ata gac cac aac acc aaa acc      300
Tyr Asp Pro Gln Val Gly Asp Tyr Phe Ile Asp His Asn Thr Lys Thr
        65                  70                  75

act cag att gag gat cct cga gta caa tgg cgg cgg gag cag gaa cat      348
Thr Gln Ile Glu Asp Pro Arg Val Gln Trp Arg Arg Glu Gln Glu His
    80                  85                  90

atg ctg aag gat tac ctg gtg gtg gcc cag gag gct ctg agt gca caa      396
Met Leu Lys Asp Tyr Leu Val Val Ala Gln Glu Ala Leu Ser Ala Gln
95                  100                 105                 110

aag gag atc tac cag gtg aag cag cag cgc ctg gag ctt gca cag cag      444
Lys Glu Ile Tyr Gln Val Lys Gln Gln Arg Leu Glu Leu Ala Gln Gln
                115                 120                 125

gag tac cag caa ctg cat gcc gtc tgg gag cat aag ctg ggc tcc cag 492
Glu Tyr Gln Gln Leu His Ala Val Trp Glu His Lys Leu Gly Ser Gln
            130                 135                 140

gtc agc ttg gtc tct ggt tca tca tcc agc tcc aag tat gac cct gag 540
Val Ser Leu Val Ser Gly Ser Ser Ser Ser Ser Lys Tyr Asp Pro Glu
        145                 150                 155

atc ctg aaa gct gaa att gcc act gca aaa tcc cgg gtc aac aag ctg 588
Ile Leu Lys Ala Glu Ile Ala Thr Ala Lys Ser Arg Val Asn Lys Leu
    160                 165                 170

aag aga gag atg gtt cac ctc cag cac gag ctg cag ttc aaa gag cgt 636
Lys Arg Glu Met Val His Leu Gln His Glu Leu Gln Phe Lys Glu Arg
175                 180                 185                 190

ggc ttt cag acc ctg aag aaa atc gat aag aaa atg tct gat gct cag 684
Gly Phe Gln Thr Leu Lys Lys Ile Asp Lys Lys Met Ser Asp Ala Gln
                195                 200                 205

ggc agc tac aaa ctg gat gaa gct cag gct gtc ttg aga gaa aca aaa 732
Gly Ser Tyr Lys Leu Asp Glu Ala Gln Ala Val Leu Arg Glu Thr Lys
            210                 215                 220

gcc atc aaa aag gct att acc tgt ggg gaa aag gaa aag caa gat ctc 780
Ala Ile Lys Lys Ala Ile Thr Cys Gly Glu Lys Glu Lys Gln Asp Leu
        225                 230                 235

att aag agc ctt gcc atg ttg aag gac ggc ttc cgc act gac agg ggg 828
Ile Lys Ser Leu Ala Met Leu Lys Asp Gly Phe Arg Thr Asp Arg Gly
    240                 245                 250

tct cac tca gac ctg tgg tcc agc agc agc tct ctg gag agt tcg agt 876
Ser His Ser Asp Leu Trp Ser Ser Ser Ser Ser Leu Glu Ser Ser Ser
255                 260                 265                 270

ttc ccg cta ccg aaa cag tac ctg gat gtg agc tcc cag aca gac atc 924
Phe Pro Leu Pro Lys Gln Tyr Leu Asp Val Ser Ser Gln Thr Asp Ile
                275                 280                 285

tcg gga agc ttc ggc atc aac agc aac aat cag ttg gca gag aag gtc 972
Ser Gly Ser Phe Gly Ile Asn Ser Asn Asn Gln Leu Ala Glu Lys Val
            290                 295                 300

aga ttg cgc ctt cga tat gaa gag gct aag aga agg atc gcc aac ctg 1020
Arg Leu Arg Leu Arg Tyr Glu Glu Ala Lys Arg Arg Ile Ala Asn Leu
        305                 310                 315

aag atc cag ctg gcc aag ctt gac agt gag gcc tgg cct ggg gtg ctg 1068
Lys Ile Gln Leu Ala Lys Leu Asp Ser Glu Ala Trp Pro Gly Val Leu
    320                 325                 330

gac tca gag agg gac cgg ctg atc ctt atc aac gag aag gag gag ctg 1116
Asp Ser Glu Arg Asp Arg Leu Ile Leu Ile Asn Glu Lys Glu Glu Leu
335                 340                 345                 350

ctg aag gag atg cgc ttc atc agc ccc cgc aag tgg acc cag ggg gag 1164
Leu Lys Glu Met Arg Phe Ile Ser Pro Arg Lys Trp Thr Gln Gly Glu

                355                 360                 365

gtg gag cag ctg gag atg gcc cgg aag cgg ctg gaa aag gac ctg cag 1212
Val Glu Gln Leu Glu Met Ala Arg Lys Arg Leu Glu Lys Asp Leu Gln
            370                 375                 380

gca gcc cgg gac acc cag agc aag gcg ctg acg gag agg tta aag tta 1260
Ala Ala Arg Asp Thr Gln Ser Lys Ala Leu Thr Glu Arg Leu Lys Leu
        385                 390                 395

aac agt aag agg aac cag ctt gtg aga gaa ctg gag gaa gcc acc cgg 1308
Asn Ser Lys Arg Asn Gln Leu Val Arg Glu Leu Glu Glu Ala Thr Arg
    400                 405                 410

cag gtg gca act ctg cac tcc cag ctg aaa agt ctc tca agc agc atg 1356
Gln Val Ala Thr Leu His Ser Gln Leu Lys Ser Leu Ser Ser Ser Met
415                 420                 425                 430

cag tcc ctg tcc tca ggc agc agc ccc gga tcc ctc acg tcc agc cgg 1404
Gln Ser Leu Ser Ser Gly Ser Ser Pro Gly Ser Leu Thr Ser Ser Arg
                435                 440                 445

ggc tcc ctg gtt gca tcc agc ctg gac tcc tcc act tca gcc agc ttc 1452
Gly Ser Leu Val Ala Ser Ser Leu Asp Ser Ser Thr Ser Ala Ser Phe
            450                 455                 460

act gac ctc tac tat gac ccc ttt gag cag ctg gac tca gag ctg cag 1500
Thr Asp Leu Tyr Tyr Asp Pro Phe Glu Gln Leu Asp Ser Glu Leu Gln
        465                 470                 475

agc aag gtg gag ttc ctg ctc ctg gag ggg gcc acc ggc ttc cgg ccc 1548
Ser Lys Val Glu Phe Leu Leu Leu Glu Gly Ala Thr Gly Phe Arg Pro
    480                 485                 490

tca ggc tgc atc acc acc atc cac gag gat gag gtg gcc aag acc cag 1596
Ser Gly Cys Ile Thr Thr Ile His Glu Asp Glu Val Ala Lys Thr Gln
495                 500                 505                 510

aag gca gag gga ggt ggc cgc ctg cag gct ctg cgt tcc ctg tct ggc 1644
Lys Ala Glu Gly Gly Gly Arg Leu Gln Ala Leu Arg Ser Leu Ser Gly
                515                 520                 525

acc cca aag tcc atg acc tcc cta tcc cca cgt tcc tct ctc tcc tcc 1692
Thr Pro Lys Ser Met Thr Ser Leu Ser Pro Arg Ser Ser Leu Ser Ser
            530                 535                 540

ccc tcc cca ccc tgt tcc cct ctc atg gct gac ccc ctc ctg gct ggt 1740
Pro Ser Pro Pro Cys Ser Pro Leu Met Ala Asp Pro Leu Leu Ala Gly
        545                 550                 555

gat gcc ttc ctc aac tcc ttg gag ttt gaa gac ccg gag ctg agt gcc 1788
Asp Ala Phe Leu Asn Ser Leu Glu Phe Glu Asp Pro Glu Leu Ser Ala
    560                 565                 570

act ctt tgt gaa ctg agc ctt ggt aac agc gcc cag gaa aga tac cgg 1836
Thr Leu Cys Glu Leu Ser Leu Gly Asn Ser Ala Gln Glu Arg Tyr Arg
575                 580                 585                 590

ctg gag gaa cca gga acg gag ggc aag cag ctg ggc caa gct gtg aat 1884
Leu Glu Glu Pro Gly Thr Glu Gly Lys Gln Leu Gly Gln Ala Val Asn
                595                 600                 605

acg gcc cag ggg tgt ggc ctg aaa gtg gcc tgt gtc tca gcc gcc gta 1932
Thr Ala Gln Gly Cys Gly Leu Lys Val Ala Cys Val Ser Ala Ala Val
            610                 615                 620

tcg gac gag tca gtg gct gga gac agt ggt gtg tac gag gct tcc gtg 1980
Ser Asp Glu Ser Val Ala Gly Asp Ser Gly Val Tyr Glu Ala Ser Val
        625                 630                 635

cag aga ctg ggt gct tca gaa gct gct gca ttt gac agt gac gaa tcg 2028
Gln Arg Leu Gly Ala Ser Glu Ala Ala Ala Phe Asp Ser Asp Glu Ser
    640                 645                 650

gaa gca gtg ggt gcg acc cga att cag att gcc ctg aag tat gat gag 2076
Glu Ala Val Gly Ala Thr Arg Ile Gln Ile Ala Leu Lys Tyr Asp Glu
655                 660                 665                 670

aag aat aag caa ttt gca ata tta atc atc cag ctg agt aac ctt tct 2124
Lys Asn Lys Gln Phe Ala Ile Leu Ile Ile Gln Leu Ser Asn Leu Ser
                675                 680                 685

gct ctg ttg cag caa caa gac cag aaa gtg aat atc cgc gtg gct gtc 2172
Ala Leu Leu Gln Gln Gln Asp Gln Lys Val Asn Ile Arg Val Ala Val
            690                 695                 700

ctt cct tgc tct gaa agc aca acc tgc ctg ttc cgg acc cgg cct ctg 2220
Leu Pro Cys Ser Glu Ser Thr Thr Cys Leu Phe Arg Thr Arg Pro Leu
        705                 710                 715

gac gcc tca gac act cta gtg ttc aat gag gtg ttc tgg gta tcc atg 2268
Asp Ala Ser Asp Thr Leu Val Phe Asn Glu Val Phe Trp Val Ser Met
    720                 725                 730

tcc tat cca gcc ctt cac cag aag acc tta aga gtc gat gtc tgt acc 2316
Ser Tyr Pro Ala Leu His Gln Lys Thr Leu Arg Val Asp Val Cys Thr
735                 740                 745                 750

acc gac agg agc cat ctg gaa gag tgc ctg gga ggc gcc cag atc agc 2364
Thr Asp Arg Ser His Leu Glu Glu Cys Leu Gly Gly Ala Gln Ile Ser
                755                 760                 765

ctg gcg gag gtc tgc cgg tct ggg gag agg tcg act cgc tgg tac aac 2412
Leu Ala Glu Val Cys Arg Ser Gly Glu Arg Ser Thr Arg Trp Tyr Asn
            770                 775                 780

ctt ctc agc tac aaa tac ttg aag aaa cag agc agg gag ctc aag cca 2460
Leu Leu Ser Tyr Lys Tyr Leu Lys Lys Gln Ser Arg Glu Leu Lys Pro
        785                 790                 795

gtg gga gtc atg gcc cct gcc tca ggg cct gcc agc acg gac gct gtg 2508
Val Gly Val Met Ala Pro Ala Ser Gly Pro Ala Ser Thr Asp Ala Val
    800                 805                 810

tct gct ctg ttg gaa cag aca gca gtg gag ctg gag aag agg cag gag 2556
Ser Ala Leu Leu Glu Gln Thr Ala Val Glu Leu Glu Lys Arg Gln Glu

815                    820                    825                    830

ggc agg agc agc aca cag aca ctg gaa gac agc tgg agg tat gag gag        2604
Gly Arg Ser Ser Thr Gln Thr Leu Glu Asp Ser Trp Arg Tyr Glu Glu
                835                    840                    845

acc agt gag aat gag gca gta gcc gag gaa gag gag gag gag gtg gag        2652
Thr Ser Glu Asn Glu Ala Val Ala Glu Glu Glu Glu Glu Glu Val Glu
            850                    855                    860

gag gag gag gga gaa gag gat gtt ttc acc gag aaa gcc tca cct gat        2700
Glu Glu Glu Gly Glu Glu Asp Val Phe Thr Glu Lys Ala Ser Pro Asp
        865                    870                    875

atg gat ggg tac cca gca tta aag gtg gac aaa gag acc aac acg gag        2748
Met Asp Gly Tyr Pro Ala Leu Lys Val Asp Lys Glu Thr Asn Thr Glu
    880                    885                    890

acc ccg gcc cca tcc ccc aca gtg gtg cga cct aag gac cgg aga gtg        2796
Thr Pro Ala Pro Ser Pro Thr Val Val Arg Pro Lys Asp Arg Arg Val
895                    900                    905                    910

ggc acc ccg tcc cag ggg cca ttt ctt cga ggg agc acc atc atc cgc        2844
Gly Thr Pro Ser Gln Gly Pro Phe Leu Arg Gly Ser Thr Ile Ile Arg
                915                    920                    925

tct aag acc ttc tcc cca gga ccc cag agc cag tac gtg tgc cgg ctg        2892
Ser Lys Thr Phe Ser Pro Gly Pro Gln Ser Gln Tyr Val Cys Arg Leu
            930                    935                    940

aat cgg agt gat agt gac agc tcc act ctg tcc aaa aag cca cct ttt        2940
Asn Arg Ser Asp Ser Asp Ser Ser Thr Leu Ser Lys Lys Pro Pro Phe
        945                    950                    955

gtt cga aac tcc ctg gag cga cgc agc gtc cgg atg aag cgg ccg tcc        2988
Val Arg Asn Ser Leu Glu Arg Arg Ser Val Arg Met Lys Arg Pro Ser
    960                    965                    970

cca ccc cca cag cct tcc tcg gtc aag tcg ctg cgc tcc gag cgt ctg        3036
Pro Pro Pro Gln Pro Ser Ser Val Lys Ser Leu Arg Ser Glu Arg Leu
975                    980                    985                    990

atc cgt acc tcg ctg gac ctg gag tta gac  ctg cag gcg aca aga  acc        3084
Ile Arg Thr Ser Leu Asp Leu Glu Leu Asp  Leu Gln Ala Thr Arg  Thr
                995                    1000                    1005

tgg cac agc caa  ttg acc cag gag atc  tcg gtg ctg aag gag  ctc        3129
Trp His Ser Gln  Leu Thr Gln Glu Ile  Ser Val Leu Lys Glu  Leu
            1010                    1015                    1020

aag gag cag ctg  gaa caa gcc aag agc  cac ggg gag aag gag  ctg        3174
Lys Glu Gln Leu  Glu Gln Ala Lys Ser  His Gly Glu Lys Glu  Leu
            1025                    1030                    1035

cca cag tgg ttg  cgt gag gac gag cgt  ttc cgc ctg ctg ctg  agg        3219
Pro Gln Trp Leu  Arg Glu Asp Glu Arg  Phe Arg Leu Leu Leu  Arg
            1040                    1045                    1050

atg ctg gag aag cgg cag atg gac cga gcg gag cac aag ggt gag 3264
Met Leu Glu Lys Arg Gln Met Asp Arg Ala Glu His Lys Gly Glu
            1055                1060                1065

ctt cag aca gac aag atg atg agg gca gct gcc aag gat gtg cac 3309
Leu Gln Thr Asp Lys Met Met Arg Ala Ala Ala Lys Asp Val His
            1070                1075                1080

agg ctc cga ggc cag agc tgt aag gaa ccc cca gaa gtt cag tct 3354
Arg Leu Arg Gly Gln Ser Cys Lys Glu Pro Pro Glu Val Gln Ser
            1085                1090                1095

ttc agg gag aag atg gca ttt ttc acc cgg cct cgg atg aat atc 3399
Phe Arg Glu Lys Met Ala Phe Phe Thr Arg Pro Arg Met Asn Ile
            1100                1105                1110

cca gct ctc tct gca gat gac gtc taa tcgccagaaa agtatttcct 3446
Pro Ala Leu Ser Ala Asp Asp Val
            1115

ttgttccact gaccaggctg tgaacattga ctgtggctaa agttatttat gtggtgttat 3506

atgaaggtac tgagtcacaa gtcctctagt gctcttgttg gtttgaagat gaaccgactt 3566

tttagtttgg gtcctactgt tgttattaaa aacagaacaa aaacaaaaca cacacacaca 3626

caaaaacaga aacaaaaaaa accagcatta aaataataag attgtatagt ttgtatattt 3686

aggagtgtat ttttgggaaa gaaaatttaa atgaactaaa gcagtattga gttgctgctc 3746

ttcttaaaat cgtttagatt ttttttggtt tgtacagctc caccttttag aggtcttact 3806

gcaataagaa gtaatgcctg ggggacggta atcctaatag gacgtcccgc acttgtcaca 3866

gtacagctaa tttttcctag ttaacatatt ttgtacaata ttaaaaaaat gcacagaaac 3926

cattgggggg gattcagagg tgcatccacg gatcttcttg agctgtgacg tgtttttatg 3986

tggctgccca acgtggagcg ggcagtgtga taggctgggt gggctaagca gcctagtcta 4046

tgtgggtgac aggccacgct ggtctcagat gcccagtgaa gccactaaca tgagtgaggg 4106

gagggctgtg gggaactcca ttcagtttta tctccatcaa taaagtggcc tttc 4160

<210> 2
<211> 1119
<212> PRT
<213> Homo sapiens

<400> 2

Met Pro Arg Pro Glu Leu Pro Leu Pro Glu Gly Trp Glu Glu Ala Arg
1               5                   10                  15

Asp Phe Asp Gly Lys Val Tyr Tyr Ile Asp His Thr Asn Arg Thr Thr
            20                  25                  30

Ser Trp Ile Asp Pro Arg Asp Arg Tyr Thr Lys Pro Leu Thr Phe Ala
        35                  40                  45

Asp Cys Ile Ser Asp Glu Leu Pro Leu Gly Trp Glu Glu Ala Tyr Asp
    50                  55                  60

Pro Gln Val Gly Asp Tyr Phe Ile Asp His Asn Thr Lys Thr Thr Gln
65                  70                  75                  80

Ile Glu Asp Pro Arg Val Gln Trp Arg Arg Glu Gln Glu His Met Leu
                85                  90                  95

Lys Asp Tyr Leu Val Val Ala Gln Glu Ala Leu Ser Ala Gln Lys Glu
            100                 105                 110

Ile Tyr Gln Val Lys Gln Gln Arg Leu Glu Leu Ala Gln Gln Glu Tyr
        115                 120                 125

Gln Gln Leu His Ala Val Trp Glu His Lys Leu Gly Ser Gln Val Ser
    130                 135                 140

Leu Val Ser Gly Ser Ser Ser Ser Ser Lys Tyr Asp Pro Glu Ile Leu
145                 150                 155                 160

Lys Ala Glu Ile Ala Thr Ala Lys Ser Arg Val Asn Lys Leu Lys Arg
                165                 170                 175

Glu Met Val His Leu Gln His Glu Leu Gln Phe Lys Glu Arg Gly Phe
            180                 185                 190

Gln Thr Leu Lys Lys Ile Asp Lys Lys Met Ser Asp Ala Gln Gly Ser
        195                 200                 205

Tyr Lys Leu Asp Glu Ala Gln Ala Val Leu Arg Glu Thr Lys Ala Ile
    210                 215                 220

Lys Lys Ala Ile Thr Cys Gly Glu Lys Glu Lys Gln Asp Leu Ile Lys
225                 230                 235                 240

Ser Leu Ala Met Leu Lys Asp Gly Phe Arg Thr Asp Arg Gly Ser His
                245                 250                 255

Ser Asp Leu Trp Ser Ser Ser Ser Ser Leu Glu Ser Ser Ser Phe Pro
            260                 265                 270

Leu Pro Lys Gln Tyr Leu Asp Val Ser Ser Gln Thr Asp Ile Ser Gly
275 280 385

Ser Phe Gly Ile Asn Ser Asn Asn Gln Leu Ala Glu Lys Val Arg Leu
    290                 295                 300

Arg Leu Arg Tyr Glu Glu Ala Lys Arg Arg Ile Ala Asn Leu Lys Ile
305                 310                 315                 320

Gln Leu Ala Lys Leu Asp Ser Glu Ala Trp Pro Gly Val Leu Asp Ser
                325                 330                 335

Glu Arg Asp Arg Leu Ile Leu Ile Asn Glu Lys Glu Glu Leu Leu Lys
            340                 345                 350

Glu Met Arg Phe Ile Ser Pro Arg Lys Trp Thr Gln Gly Glu Val Glu
        355                 360                 365

Gln Leu Glu Met Ala Arg Lys Arg Leu Glu Lys Asp Leu Gln Ala Ala
    370                 375                 380

Arg Asp Thr Gln Ser Lys Ala Leu Thr Glu Arg Leu Lys Leu Asn Ser
385                 390                 395                 400

Lys Arg Asn Gln Leu Val Arg Glu Leu Glu Glu Ala Thr Arg Gln Val
                405                 410                 415

Ala Thr Leu His Ser Gln Leu Lys Ser Leu Ser Ser Ser Met Gln Ser
            420                 425                 430

Leu Ser Ser Gly Ser Ser Pro Gly Ser Leu Thr Ser Ser Arg Gly Ser
        435                 440                 445

Leu Val Ala Ser Ser Leu Asp Ser Ser Thr Ser Ala Ser Phe Thr Asp
    450                 455                 460

Leu Tyr Tyr Asp Pro Phe Glu Gln Leu Asp Ser Glu Leu Gln Ser Lys
465                 470                 475                 480

Val Glu Phe Leu Leu Leu Glu Gly Ala Thr Gly Phe Arg Pro Ser Gly
                485                 490                 495

Cys Ile Thr Thr Ile His Glu Asp Glu Val Ala Lys Thr Gln Lys Ala
            500                 505                 510

Glu Gly Gly Gly Arg Leu Gln Ala Leu Arg Ser Leu Ser Gly Thr Pro
        515                 520                 525

Lys Ser Met Thr Ser Leu Ser Pro Arg Ser Ser Leu Ser Ser Pro Ser
    530                 535                 540

Pro Pro Cys Ser Pro Leu Met Ala Asp Pro Leu Leu Ala Gly Asp Ala
545                 550                 555                 560

Phe Leu Asn Ser Leu Glu Phe Glu Asp Pro Glu Leu Ser Ala Thr Leu
                565                 570                 575

Cys Glu Leu Ser Leu Gly Asn Ser Ala Gln Glu Arg Tyr Arg Leu Glu
            580                 585                 590

Glu Pro Gly Thr Glu Gly Lys Gln Leu Gly Gln Ala Val Asn Thr Ala
        595                 600                 605

Gln Gly Cys Gly Leu Lys Val Ala Cys Val Ser Ala Ala Val Ser Asp
    610                 615                 620

Glu Ser Val Ala Gly Asp Ser Gly Val Tyr Glu Ala Ser Val Gln Arg
625                 630                 635                 640

Leu Gly Ala Ser Glu Ala Ala Ala Phe Asp Ser Asp Glu Ser Glu Ala
                645                 650                 655

Val Gly Ala Thr Arg Ile Gln Ile Ala Leu Lys Tyr Asp Glu Lys Asn
            660                 665                 670

Lys Gln Phe Ala Ile Leu Ile Ile Gln Leu Ser Asn Leu Ser Ala Leu
        675                 680                 685

Leu Gln Gln Gln Asp Gln Lys Val Asn Ile Arg Val Ala Val Leu Pro
    690                 695                 700

Cys Ser Glu Ser Thr Thr Cys Leu Phe Arg Thr Arg Pro Leu Asp Ala
705                 710                 715                 720

Ser Asp Thr Leu Val Phe Asn Glu Val Phe Trp Val Ser Met Ser Tyr
                725                 730                 735

Pro Ala Leu His Gln Lys Thr Leu Arg Val Asp Val Cys Thr Thr Asp
            740                 745                 750

Arg Ser His Leu Glu Glu Cys Leu Gly Gly Ala Gln Ile Ser Leu Ala
        755                 760                 765

Glu Val Cys Arg Ser Gly Glu Arg Ser Thr Arg Trp Tyr Asn Leu Leu
    770                 775                 780

Ser Tyr Lys Tyr Leu Lys Lys Gln Ser Arg Glu Leu Lys Pro Val Gly
785                 790                 795                 800

Val Met Ala Pro Ala Ser Gly Pro Ala Ser Thr Asp Ala Val Ser Ala
                805                 810                 815

Leu Leu Glu Gln Thr Ala Val Glu Leu Glu Lys Arg Gln Glu Gly Arg
            820                 825                 830

Ser Ser Thr Gln Thr Leu Glu Asp Ser Trp Arg Tyr Glu Glu Thr Ser
        835                 840                 845

Glu Asn Glu Ala Val Ala Glu Glu Glu Glu Glu Glu Val Glu Glu Glu
    850                 855                 860

Glu Gly Glu Glu Asp Val Phe Thr Glu Lys Ala Ser Pro Asp Met Asp
865                 870                 875                 880

Gly Tyr Pro Ala Leu Lys Val Asp Lys Glu Thr Asn Thr Glu Thr Pro
                885                 890                 895

Ala Pro Ser Pro Thr Val Val Arg Pro Lys Asp Arg Arg Val Gly Thr
            900                 905                 910

Pro Ser Gln Gly Pro Phe Leu Arg Gly Ser Thr Ile Ile Arg Ser Lys
        915                 920                 925

Thr Phe Ser Pro Gly Pro Gln Ser Gln Tyr Val Cys Arg Leu Asn Arg
    930                 935                 940

Ser Asp Ser Asp Ser Ser Thr Leu Ser Lys Lys Pro Pro Phe Val Arg
945                 950                 955                 960

Asn Ser Leu Glu Arg Arg Ser Val Arg Met Lys Arg Pro Ser Pro Pro
                965                 970                 975

Pro Gln Pro Ser Ser Val Lys Ser Leu Arg Ser Glu Arg Leu Ile Arg
            980                 985                 990

Thr Ser Leu Asp Leu Glu Leu Asp  Leu Gln Ala Thr Arg  Thr Trp His
        995                 1000                 1005

Ser Gln  Leu Thr Gln Glu Ile  Ser Val Leu Lys Glu  Leu Lys Glu
    1010                 1015                 1020

Gln Leu  Glu Gln Ala Lys Ser  His Gly Glu Lys Glu  Leu Pro Gln
    1025                 1030                 1035

Trp Leu  Arg Glu Asp Glu Arg  Phe Arg Leu Leu Leu  Arg Met Leu
    1040                 1045                 1050

Glu Lys  Arg Gln Met Asp Arg  Ala Glu His Lys Gly  Glu Leu Gln
    1055                 1060                 1065

Thr Asp  Lys Met Met Arg Ala  Ala Ala Lys Asp Val  His Arg Leu
    1070                 1075                 1080

Arg Gly  Gln Ser Cys Lys Glu  Pro Pro Glu Val Gln  Ser Phe Arg
    1085                 1090                 1095

Glu Lys  Met Ala Phe Phe Thr  Arg Pro Arg Met Asn  Ile Pro Ala
    1100                 1105                 1110

Leu Ser  Ala Asp Asp Val
    1115

<210>  3
<211>  4467
<212>  DNA
<213>  Mus musculus

<220>
<221>  CDS
<222>  (379)..(3693)

<400>  3
   cgcgcggcgg ggctccggca gaggctgcgg gcggcggcgg cggcggcggc ggcgtggagc      60
   gcagcgcgca gcggcagggg caggcaggcg gcggctcggg ccgctctcct ggctggctgg 120
   ccgcggactc gggccggctg ggcgccgcgg cgggcgcaca tggcagcgtg agaggcgggc 180
   ggcgtggggc tggggccgca gggccgcatg gacagaggcg ccacccaggc cggcccccgt 240
   tagggccccc gatccgtggg cgccactccc cgatcatgct gcctcggcgg ccgcctggac 300
   ccaaagcggc cagcccgagc cgctgagccc cgtggcggct gcgagctgca tgggggagcg 360
   cgggccaggc tcgggaag atg ccc cgg ccg gag ttg ccc ctg ccg gag ggc 411
   Met Pro Arg Pro Glu Leu Pro Leu Pro Glu Gly
   1 5 10
   tgg gag gag gcg cgc gac ttc gac ggc aag gtc tac tac ata gat cac 459
   Trp Glu Glu Ala Arg Asp Phe Asp Gly Lys Val Tyr Tyr Ile Asp His
   15 20 25
   agg aac cgc acc acc agc tgg atc gac ccg cgg gac agg tac acc aaa 507
   Arg Asn Arg Thr Thr Ser Trp Ile Asp Pro Arg Asp Arg Tyr Thr Lys
   30 35 40
   cca ctc acc ttc gct gac tgc atc agt gat gag tta ccg ctg gga tgg 555
   Pro Leu Thr Phe Ala Asp Cys Ile Ser Asp Glu Leu Pro Leu Gly Trp
   45 50 55
   gaa gaa gca tac gac cca cag gtc gga gat tac ttc ata gac cac aat 603
   Glu Glu Ala Tyr Asp Pro Gln Val Gly Asp Tyr Phe Ile Asp His Asn
   60 65 70 75
   acc aaa acc act cag atc gag gat cca agg gtg caa tgg cgg cgg gaa 651
   Thr Lys Thr Thr Gln Ile Glu Asp Pro Arg Val Gln Trp Arg Arg Glu
   80 85 90
   cag gag cac atg ctg aag gat tac ctg gtg gtg gct cag gaa gct ctg 699
   Gln Glu His Met Leu Lys Asp Tyr Leu Val Val Ala Gln Glu Ala Leu
   95 100 105
   agt gca caa aag gag atc tac cag gtg aag cag cag cgc ctg gag ctg 747
   Ser Ala Gln Lys Glu Ile Tyr Gln Val Lys Gln Gln Arg Leu Glu Leu
   110 115 120
   gca cag cag gag tat cag cag ttg cat gcc gtc tgg gag cat aag ctg 795
   Ala Gln Gln Glu Tyr Gln Gln Leu His Ala Val Trp Glu His Lys Leu
   125 130 135
   ggc tcc cag gtt agc ttg gtc tct gga tcc tcg tcc agc tcc aag tat 843
   Gly Ser Gln Val Ser Leu Val Ser Gly Ser Ser Ser Ser Ser Lys Tyr
   140 145 150 155
   gac ccc gag atc ctg aaa gct gaa att gcc act gca aag tcc cgg gtc 891
   Asp Pro Glu Ile Leu Lys Ala Glu Ile Ala Thr Ala Lys Ser Arg Val
   160 165 170
   aac aag ctg aag aga gag atg gtt cac cta cag cat gag ctg cag ttc 939
   Asn Lys Leu Lys Arg Glu Met Val His Leu Gln His Glu Leu Gln Phe
   175 180 185
   aag gag cgt ggc ttt cag acg ctg aag aaa att gat gag aga atg tca 987
   Lys Glu Arg Gly Phe Gln Thr Leu Lys Lys Ile Asp Glu Arg Met Ser
   190 195 200
   gac gca cag ggt ggc tac aaa ctg gat gag gcc cag gct gtg ctg agg 1035
   Asp Ala Gln Gly Gly Tyr Lys Leu Asp Glu Ala Gln Ala Val Leu Arg
   205 210 215
   gaa acc aaa gcc atc aag aaa gcc atc acc tgc gga gaa aag gag aaa 1083
   Glu Thr Lys Ala Ile Lys Lys Ala Ile Thr Cys Gly Glu Lys Glu Lys
   220 225 230 235
   caa gac ctc att aag agt ctc gcc atg ctg aag gat ggc ttc cgg aca 1131
   Gln Asp Leu Ile Lys Ser Leu Ala Met Leu Lys Asp Gly Phe Arg Thr
   240 245 250
   gac aga ggg tca cac tcc gac cta tgg tcc agc agc agc tct ctg gag 1179
   Asp Arg Gly Ser His Ser Asp Leu Trp Ser Ser Ser Ser Ser Leu Glu
   255 260 265
   agt tca agc ttc ccg atg cca aag cag ttc ctg gac gtg agc tcg cag 1227
   Ser Ser Ser Phe Pro Met Pro Lys Gln Phe Leu Asp Val Ser Ser Gln
   270 275 280
   aca gac atc tct ggg agt ttc agc acc agc agc aac aat cag ttg gcc 1275
   Thr Asp Ile Ser Gly Ser Phe Ser Thr Ser Ser Asn Asn Gln Leu Ala
   285 290 295
   gag aag gtc aga ttg cgc ctt cgg tat gaa gag gcg aag aga agg atc 1323
   Glu Lys Val Arg Leu Arg Leu Arg Tyr Glu Glu Ala Lys Arg Arg Ile
   300 305 310 315
   gcc aac ctc aag atc cag ctg gcc aaa ctt gac agt gag gcc tgg cct 1371
   Ala Asn Leu Lys Ile Gln Leu Ala Lys Leu Asp Ser Glu Ala Trp Pro
   320 325 330
   ggg gtg cta gac tca gag agg gac cga ctg atc ctc atc aat gag aag 1419
   Gly Val Leu Asp Ser Glu Arg Asp Arg Leu Ile Leu Ile Asn Glu Lys
   335 340 345
   gag gaa ctt ctg aag gaa atg cgc ttc atc agc ccc cgg aaa tgg acc 1467
   Glu Glu Leu Leu Lys Glu Met Arg Phe Ile Ser Pro Arg Lys Trp Thr
   350 355 360
   cag gga gag gtg gag caa ctg gag atg gcc cgg agg cga ctt gag aag 1515
   Gln Gly Glu Val Glu Gln Leu Glu Met Ala Arg Arg Arg Leu Glu Lys
   365 370 375
   gac tta cag gca gcc cgg gac acc cag agc aag gcg ctg act gag agg 1563
   Asp Leu Gln Ala Ala Arg Asp Thr Gln Ser Lys Ala Leu Thr Glu Arg
   380 385 390 395
   ctg aag tta aat agc aag agg aac cag ctg gtg cga gaa ctg gag gaa 1611
   Leu Lys Leu Asn Ser Lys Arg Asn Gln Leu Val Arg Glu Leu Glu Glu
   400 405 410
   gcc act cgg cag gtg gct acc ctg cac tcc cag ttg aaa agc ctc tca 1659
   Ala Thr Arg Gln Val Ala Thr Leu His Ser Gln Leu Lys Ser Leu Ser
               415                 420                 425
   agc agc atg cag tcc ctg tct tca ggc agt agc cct ggg tca ctc acc      1707
   Ser Ser Met Gln Ser Leu Ser Ser Gly Ser Ser Pro Gly Ser Leu Thr
           430                 435                 440
   tcc agc cgg ggc tcc ctg gcc gcc tct agc ctg gac tcc tcc acc tca      1755
   Ser Ser Arg Gly Ser Leu Ala Ala Ser Ser Leu Asp Ser Ser Thr Ser
       445                 450                 455
   gcc agc ttc act gac ctc tat tat gac ccg ttc gag cag ttg gac tcg      1803
   Ala Ser Phe Thr Asp Leu Tyr Tyr Asp Pro Phe Glu Gln Leu Asp Ser
   460                 465                 470                 475
   gag ctc cag agc aag gtg gag ctg ctg ttc ctg gag ggt gcc acg ggc      1851
   Glu Leu Gln Ser Lys Val Glu Leu Leu Phe Leu Glu Gly Ala Thr Gly
                   480                 485                 490
   ttc cgg ccc tca ggc tgc atc acc acc atc cac gag gat gag gtg gcc      1899
   Phe Arg Pro Ser Gly Cys Ile Thr Thr Ile His Glu Asp Glu Val Ala
               495                 500                 505
   aag acc cag aag gca gag gga ggc agc cgc ctg cag gcc ctg cgc tcc      1947
   Lys Thr Gln Lys Ala Glu Gly Gly Ser Arg Leu Gln Ala Leu Arg Ser
           510                 515                 520
   cta tct ggc act cca agg tcc atg acc tcc ctg tct cca cgc tca tct      1995
   Leu Ser Gly Thr Pro Arg Ser Met Thr Ser Leu Ser Pro Arg Ser Ser
       525                 530                 535
   ctc tcc tcc cca tcc cct ccc tgt tcc cct ctc atc act gat ccc ctc      2043
   Leu Ser Ser Pro Ser Pro Pro Cys Ser Pro Leu Ile Thr Asp Pro Leu
   540                 545                 550                 555
   ctg act gga gat gcc ttc ctg gcc ccg ctg gag ttt gaa gac aca gag      2091
   Leu Thr Gly Asp Ala Phe Leu Ala Pro Leu Glu Phe Glu Asp Thr Glu
                   560                 565                 570
   ctg agt acc act ctc tgt gag ctg aac ctc ggc ggt agc ggc acc cag      2139
   Leu Ser Thr Thr Leu Cys Glu Leu Asn Leu Gly Gly Ser Gly Thr Gln
               575                 580                 585
   gaa aga tac cgg ctg gag gaa cca gga cct gag ggc aag cca ctg ggc      2187
   Glu Arg Tyr Arg Leu Glu Glu Pro Gly Pro Glu Gly Lys Pro Leu Gly
           590                 595                 600
   caa gct gca agt gtg gcc cca gga tgt ggc ctc aaa gta gcc tgt gtg      2235
   Gln Ala Ala Ser Val Ala Pro Gly Cys Gly Leu Lys Val Ala Cys Val
       605                 610                 615
   tcc gct gca gtg tcc gac gag tca gta gcc ggg gac agt ggc gtg tat      2283
   Ser Ala Ala Val Ser Asp Glu Ser Val Ala Gly Asp Ser Gly Val Tyr
   620                 625                 630                 635
   gag gct tca gca cag aga cca ggt act tct gaa gct gcc gca ttc gac      2331
   Glu Ala Ser Ala Gln Arg Pro Gly Thr Ser Glu Ala Ala Ala Phe Asp
                   640                 645                 650
   agt gat gaa tcg gaa gct gtg ggt gcc act cgg gtt cag att gcc ctg     2379
   Ser Asp Glu Ser Glu Ala Val Gly Ala Thr Arg Val Gln Ile Ala Leu
               655                 660                 665
   aaa tat gat gag aag aat aag caa ttt gca ata tta atc att cag ctg     2427
   Lys Tyr Asp Glu Lys Asn Lys Gln Phe Ala Ile Leu Ile Ile Gln Leu
           670                 675                 680
   agt cac ctc tct gct ctg tca cta caa caa gac cag aaa gtg aac atc     2475
   Ser His Leu Ser Ala Leu Ser Leu Gln Gln Asp Gln Lys Val Asn Ile
       685                 690                 695
   cgt gta gcc atc ctg cct tgc tct gaa agc agc acc tgc ttg ttc aga     2523
   Arg Val Ala Ile Leu Pro Cys Ser Glu Ser Ser Thr Cys Leu Phe Arg
   700                 705                 710                 715
   acc cgg cct ctg gac tcg gca aat acc ctc gtg ttc aat gag gca ttc     2571
   Thr Arg Pro Leu Asp Ser Ala Asn Thr Leu Val Phe Asn Glu Ala Phe
                   720                 725                 730
   tgg gtg tcc atc tcc tac cca gcc ctt cac caa aag acc tta cga gtc     2619
   Trp Val Ser Ile Ser Tyr Pro Ala Leu His Gln Lys Thr Leu Arg Val
               735                 740                 745
   gat gtc tgc acc act gac agg agc cat aca gag gag tgt ctg gga ggt     2667
   Asp Val Cys Thr Thr Asp Arg Ser His Thr Glu Glu Cys Leu Gly Gly
           750                 755                 760
   gcc cag atc agc ctg gcc gag gtc tgc cgg tct ggg gag agg tca acg     2715
   Ala Gln Ile Ser Leu Ala Glu Val Cys Arg Ser Gly Glu Arg Ser Thr
       765                 770                 775
   cgc tgg tac aat ctc ctg agc tac aaa tac ttg aag aag cag tgc agg     2763
   Arg Trp Tyr Asn Leu Leu Ser Tyr Lys Tyr Leu Lys Lys Gln Cys Arg
   780                 785                 790                 795
   gag ccc cag cca aca gaa gct cca ggg cca gac cat gtg gac gct gtg     2811
   Glu Pro Gln Pro Thr Glu Ala Pro Gly Pro Asp His Val Asp Ala Val
                   800                 805                 810
   tct gcc ttg cta gaa cag aca gcc gtg gag cta gag aag agg cag gag     2859
   Ser Ala Leu Leu Glu Gln Thr Ala Val Glu Leu Glu Lys Arg Gln Glu
               815                 820                 825
   gga aga agc agc tcc cag acg ctg gag ggc agc tgg acg tac gag gaa     2907
   Gly Arg Ser Ser Ser Gln Thr Leu Glu Gly Ser Trp Thr Tyr Glu Glu
           830                 835                 840
   gag gcc agc gag aat gag gca gtc gct gag gag gaa gag gag gga gag     2955
   Glu Ala Ser Glu Asn Glu Ala Val Ala Glu Glu Glu Glu Glu Gly Glu
       845                 850                 855
   gaa gat gtc ttc act gaa aag gtc tct ccc gag gca gag gag tgc cca     3003
   Glu Asp Val Phe Thr Glu Lys Val Ser Pro Glu Ala Glu Glu Cys Pro
   860                 865                 870                 875
   gca tta aag gtg gac aga gag acc aat act gac agc gtg gcc cca tcc     3051
   Ala Leu Lys Val Asp Arg Glu Thr Asn Thr Asp Ser Val Ala Pro Ser
   880 885 890
   ccc aca gtg gtg cgc cct aag gac cgg agg gtg ggt gcc ccg tcg aca 3099
   Pro Thr Val Val Arg Pro Lys Asp Arg Arg Val Gly Ala Pro Ser Thr
   895 900 905
   ggg cca ttt ctt cga ggg aat acc atc atc cgc tcc aag acc ttc tcg 3147
   Gly Pro Phe Leu Arg Gly Asn Thr Ile Ile Arg Ser Lys Thr Phe Ser
   910 915 920
   cct gga ccc cag agc cag tat gtg tgt cgg cta aac cgc agt gac agt 3195
   Pro Gly Pro Gln Ser Gln Tyr Val Cys Arg Leu Asn Arg Ser Asp Ser
   925 930 935
   gat agt tct acg ctg tcc aag aag cca cct ttt gtg cga aac tcc ctg 3243
   Asp Ser Ser Thr Leu Ser Lys Lys Pro Pro Phe Val Arg Asn Ser Leu
   940 945 950 955
   gag cgg cgc agc gtc agg atg aag cgg cct tcc tcc gtc aag tcc ctg 3291
   Glu Arg Arg Ser Val Arg Met Lys Arg Pro Ser Ser Val Lys Ser Leu
   960 965 970
   cgc aca gag cgt ctg atc cgc acc tcg ctg gac cta gaa ctc gac ctg 3339
   Arg Thr Glu Arg Leu Ile Arg Thr Ser Leu Asp Leu Glu Leu Asp Leu
   975 980 985
   cag gcc acg agg acc tgg cac agc cag ctg act cag gag atc tca gtg 3387
   Gln Ala Thr Arg Thr Trp His Ser Gln Leu Thr Gln Glu Ile Ser Val
   990 995 1000
   ctg aag gag ctg aag gag cat ttg gag caa gcc aag aac cac ggg 3432
   Leu Lys Glu Leu Lys Glu His Leu Glu Gln Ala Lys Asn His Gly
   1005 1010 1015
   gag aag gag ctg cca cag tgg ctg cgt gaa gac gag cgc ttc cgc 3477
   Glu Lys Glu Leu Pro Gln Trp Leu Arg Glu Asp Glu Arg Phe Arg
   1020 1025 1030
   ttg cta ctg agg atg ctg gag aag aag gtg gat cgt ggg gag cac 3522
   Leu Leu Leu Arg Met Leu Glu Lys Lys Val Asp Arg Gly Glu His
   1035 1040 1045
   aag agt gag ttg caa gcc gac aag atg atg aga gct gct gcc aag 3567
   Lys Ser Glu Leu Gln Ala Asp Lys Met Met Arg Ala Ala Ala Lys
   1050 1055 1060
   gat gtg cac agg ctt cga ggc cag agc tgt aag gag ccc cca gaa 3612
   Asp Val His Arg Leu Arg Gly Gln Ser Cys Lys Glu Pro Pro Glu
   1065 1070 1075
   gtg cag tct ttc agg gag aaa atg gca ttt ttc acc cgg cct cgg 3657
   Val Gln Ser Phe Arg Glu Lys Met Ala Phe Phe Thr Arg Pro Arg
   1080 1085 1090
   atg aac atc cca gct ctc tct gca gat gac gtc taa ccaccagaaa 3703
   Met Asn Ile Pro Ala Leu Ser Ala Asp Asp Val
   1095 1100
   agtatttcct ttgtctcgtg cgcgcgcgtg cagaccaagc tgtgaaggct gactgtgact 3763
   aatgttattt atgaggcatt atatgaaggt actgagtcac aagtccccga gcgctcttgt 3823
   tggtttgaag acagattttt tttttaaatt tcaggttttc ttgttattat ttaaaaaaaa 3883
   aaatgacaaa gcaaccataa caacaaaaac aaaaaccaaa caaacaaaaa aaccagcatt 3943
   aaaatgacaa gattgtatag tttgtatatt taggaatgta tttttgggaa agaatttaaa 4003
   tgaactaaag cagcattaag tggctgctag tcttaaaact gtctagagtt ttctttttgt 4063
   acagcaacaa ctttcagagg tttccctgca ataataaaaa aaaaaaaagt tgtatttgag 4123
   gggcgacaat cctcaaagga ggtcccgcgg gtggcacagc acagctagcc tgccctggtt 4183
   aacgttattt tgtacaataa tacaaagaca aggatgtaca gtaatcgtgg gggaattcga 4243
   agactgtccg cggatctttg tgagctgtga tgtgtctttc atgtggccac gtgaccgtga 4303
   ccggaagcag gtagcctgga gctggctgaa ccgcccggac tggtgacagg ccacgcccag 4363
   gccacgccca tctcagatgc ccagtgacgc agctgtggga gtgaggggag ggctgcggag 4423
   aaccgcattt ggatctatct ctaacaataa agtggccttt taag 4467

<210> 4
<211> 1104
<212> PRT
<213> Mus musculus

<400> 4

Met Pro Arg Pro Glu Leu Pro Leu Pro Glu Gly Trp Glu Glu Ala Arg
1               5                   10                  15

Asp Phe Asp Gly Lys Val Tyr Tyr Ile Asp His Arg Asn Arg Thr Thr
            20                  25                  30

Ser Trp Ile Asp Pro Arg Asp Arg Tyr Thr Lys Pro Leu Thr Phe Ala
        35                  40                  45

Asp Cys Ile Ser Asp Glu Leu Pro Leu Gly Trp Glu Glu Ala Tyr Asp
    50                  55                  60

Pro Gln Val Gly Asp Tyr Phe Ile Asp His Asn Thr Lys Thr Thr Gln
65                  70                  75                  80

Ile Glu Asp Pro Arg Val Gln Trp Arg Arg Glu Gln Glu His Met Leu
                85                  90                  95

Lys Asp Tyr Leu Val Val Ala Gln Glu Ala Leu Ser Ala Gln Lys Glu
            100                 105                 110

Ile Tyr Gln Val Lys Gln Gln Arg Leu Glu Leu Ala Gln Gln Glu Tyr
        115                 120                 125

Gln Gln Leu His Ala Val Trp Glu His Lys Leu Gly Ser Gln Val Ser
    130                 135                 140

Leu Val Ser Gly Ser Ser Ser Ser Ser Lys Tyr Asp Pro Glu Ile Leu
145                 150                 155                 160

Lys Ala Glu Ile Ala Thr Ala Lys Ser Arg Val Asn Lys Leu Lys Arg
                165                 170                 175

Glu Met Val His Leu Gln His Glu Leu Gln Phe Lys Glu Arg Gly Phe
100 105 190

Gln Thr Leu Lys Lys Ile Asp Glu Arg Met Ser Asp Ala Gln Gly Gly
        195                 200                 205

Tyr Lys Leu Asp Glu Ala Gln Ala Val Leu Arg Glu Thr Lys Ala Ile
    210                 215                 220

Lys Lys Ala Ile Thr Cys Gly Glu Lys Glu Lys Gln Asp Leu Ile Lys
225                 230                 235                 240

Ser Leu Ala Met Leu Lys Asp Gly Phe Arg Thr Asp Arg Gly Ser His
                245                 250                 255

Ser Asp Leu Trp Ser Ser Ser Ser Ser Leu Glu Ser Ser Ser Phe Pro
            260                 265                 270

Met Pro Lys Gln Phe Leu Asp Val Ser Ser Gln Thr Asp Ile Ser Gly
        275                 280                 285

Ser Phe Ser Thr Ser Ser Asn Asn Gln Leu Ala Glu Lys Val Arg Leu
    290                 295                 300

Arg Leu Arg Tyr Glu Glu Ala Lys Arg Arg Ile Ala Asn Leu Lys Ile
305                 310                 315                 320

Gln Leu Ala Lys Leu Asp Ser Glu Ala Trp Pro Gly Val Leu Asp Ser

                325                 330                 335

Glu Arg Asp Arg Leu Ile Leu Ile Asn Glu Lys Glu Glu Leu Leu Lys
            340                 345                 350

Glu Met Arg Phe Ile Ser Pro Arg Lys Trp Thr Gln Gly Glu Val Glu
        355                 360                 365

Gln Leu Glu Met Ala Arg Arg Arg Leu Glu Lys Asp Leu Gln Ala Ala
    370                 375                 380

Arg Asp Thr Gln Ser Lys Ala Leu Thr Glu Arg Leu Lys Leu Asn Ser
385                 390                 395                 400

Lys Arg Asn Gln Leu Val Arg Glu Leu Glu Glu Ala Thr Arg Gln Val
                405                 410                 415

Ala Thr Leu His Ser Gln Leu Lys Ser Leu Ser Ser Ser Met Gln Ser
            420                 425                 430

Leu Ser Ser Gly Ser Ser Pro Gly Ser Leu Thr Ser Ser Arg Gly Ser
        435                 440                 445

Leu Ala Ala Ser Ser Leu Asp Ser Ser Thr Ser Ala Ser Phe Thr Asp
    450                 455                 460

Leu Tyr Tyr Asp Pro Phe Glu Gln Leu Asp Ser Glu Leu Gln Ser Lys
465                 470                 475                 480

Val Glu Leu Leu Phe Leu Glu Gly Ala Thr Gly Phe Arg Pro Ser Gly
                485                 490                 495

Cys Ile Thr Thr Ile His Glu Asp Glu Val Ala Lys Thr Gln Lys Ala
            500                 505                 510

Glu Gly Gly Ser Arg Leu Gln Ala Leu Arg Ser Leu Ser Gly Thr Pro
        515                 520                 525

Arg Ser Met Thr Ser Leu Ser Pro Arg Ser Ser Leu Ser Ser Pro Ser
    530                 535                 540

Pro Pro Cys Ser Pro Leu Ile Thr Asp Pro Leu Leu Thr Gly Asp Ala
545                 550                 555                 560

Phe Leu Ala Pro Leu Glu Phe Glu Asp Thr Glu Leu Ser Thr Thr Leu
                565                 570                 575

Cys Glu Leu Asn Leu Gly Gly Ser Gly Thr Gln Glu Arg Tyr Arg Leu
            580                 585                 590

Glu Glu Pro Gly Pro Glu Gly Lys Pro Leu Gly Gln Ala Ala Ser Val
        595                 600                 605

Ala Pro Gly Cys Gly Leu Lys Val Ala Cys Val Ser Ala Ala Val Ser
    610                 615                 620

Asp Glu Ser Val Ala Gly Asp Ser Gly Val Tyr Glu Ala Ser Ala Gln
625                 630                 635                 640

Arg Pro Gly Thr Ser Glu Ala Ala Ala Phe Asp Ser Asp Glu Ser Glu
                645                 650                 655

Ala Val Gly Ala Thr Arg Val Gln Ile Ala Leu Lys Tyr Asp Glu Lys
            660                 665                 670

Asn Lys Gln Phe Ala Ile Leu Ile Ile Gln Leu Ser His Leu Ser Ala
        675                 680                 685

Leu Ser Leu Gln Gln Asp Gln Lys Val Asn Ile Arg Val Ala Ile Leu
    690                 695                 700

Pro Cys Ser Glu Ser Ser Thr Cys Leu Phe Arg Thr Arg Pro Leu Asp
705                 710                 715                 720

Ser Ala Asn Thr Leu Val Phe Asn Glu Ala Phe Trp Val Ser Ile Ser
                725                 730                 735

Tyr Pro Ala Leu His Gln Lys Thr Leu Arg Val Asp Val Cys Thr Thr
            740                 745                 750

Asp Arg Ser His Thr Glu Glu Cys Leu Gly Gly Ala Gln Ile Ser Leu
        755                 760                 765

Ala Glu Val Cys Arg Ser Gly Glu Arg Ser Thr Arg Trp Tyr Asn Leu
    770                 775                 780

Leu Ser Tyr Lys Tyr Leu Lys Lys Gln Cys Arg Glu Pro Gln Pro Thr

785                 790                 795                 800

Glu Ala Pro Gly Pro Asp His Val Asp Ala Val Ser Ala Leu Leu Glu
                805                 810                 815

Gln Thr Ala Val Glu Leu Glu Lys Arg Gln Glu Gly Arg Ser Ser Ser
            820                 825                 830

Gln Thr Leu Glu Gly Ser Trp Thr Tyr Glu Glu Glu Ala Ser Glu Asn
        835                 840                 845

Glu Ala Val Ala Glu Glu Glu Glu Glu Gly Glu Glu Asp Val Phe Thr
    850                 855                 860

Glu Lys Val Ser Pro Glu Ala Glu Glu Cys Pro Ala Leu Lys Val Asp
865                 870                 875                 880

Arg Glu Thr Asn Thr Asp Ser Val Ala Pro Ser Pro Thr Val Val Arg
                885                 890                 895

Pro Lys Asp Arg Arg Val Gly Ala Pro Ser Thr Gly Pro Phe Leu Arg
            900                 905                 910

Gly Asn Thr Ile Ile Arg Ser Lys Thr Phe Ser Pro Gly Pro Gln Ser
        915                 920                 925

Gln Tyr Val Cys Arg Leu Asn Arg Ser Asp Ser Asp Ser Ser Thr Leu
    930                 935                 940

Ser Lys Lys Pro Pro Phe Val Arg Asn Ser Leu Glu Arg Arg Ser Val
945                 950                 955                 960

Arg Met Lys Arg Pro Ser Ser Val Lys Ser Leu Arg Thr Glu Arg Leu
                965                 970                 975

Ile Arg Thr Ser Leu Asp Leu Glu Leu Asp Leu Gln Ala Thr Arg Thr
            980                 985                 990

Trp His Ser Gln Leu Thr Gln Glu  Ile Ser Val Leu Lys  Glu Leu Lys
        995                 1000                 1005

Glu His  Leu Glu Gln Ala Lys  Asn His Gly Glu Lys  Glu Leu Pro
    1010                 1015                 1020

Gln Trp Leu Arg Glu Asp Glu Arg Phe Arg Leu Leu Leu Arg Met
    1025                1030                1035

Leu Glu Lys Lys Val Asp Arg Gly Glu His Lys Ser Glu Leu Gln
    1040                1045                1050

Ala Asp Lys Met Met Arg Ala Ala Ala Lys Asp Val His Arg Leu
    1055                1060                1065

Arg Gly Gln Ser Cys Lys Glu Pro Pro Glu Val Gln Ser Phe Arg
    1070                1075                1080

Glu Lys Met Ala Phe Phe Thr Arg Pro Arg Met Asn Ile Pro Ala
    1085                1090                1095

Leu Ser Ala Asp Asp Val
    1100

<210> 5
<211> 4181
<212> DNA
<213> Rattus norvegicus

<220>
<221> CDS
<222> (93)..(3419)

<400> 5
gcggccgcct ggacccaaag cggccggcct gagccgctga gccccgtggc ggccgcgagc 60

tgcatggggg agcgcgggcc aggctcggga ag atg ccc cgg ccg gag ttg ccc 113
Met Pro Arg Pro Glu Leu Pro
1               5

ctg ccg gag ggc tgg gag gag gcg cgc gac ttc gac ggc aag gtc tac 161
Leu Pro Glu Gly Trp Glu Glu Ala Arg Asp Phe Asp Gly Lys Val Tyr
        10                  15                  20

tac ata gat cac agg aac cgc acc acc agc tgg atc gac ccg cgg gac 209
Tyr Ile Asp His Arg Asn Arg Thr Thr Ser Trp Ile Asp Pro Arg Asp
    25                  30                  35

agg tac acc aaa cca ctc acc ttt gct gac tgc att agc gac gag ttg 257
Arg Tyr Thr Lys Pro Leu Thr Phe Ala Asp Cys Ile Ser Asp Glu Leu
40                  45                  50                  55

ccg ctg gga tgg gaa gaa gcg tat gat cca cag gtt gga gat tac ttc 305
Pro Leu Gly Trp Glu Glu Ala Tyr Asp Pro Gln Val Gly Asp Tyr Phe
                60                  65                  70

ata gac cac aat acc aaa acc act cag atc gag gat ccg agg gtg caa 353
Ile Asp His Asn Thr Lys Thr Thr Gln Ile Glu Asp Pro Arg Val Gln
            75                  80                  85

tgg cgg cgg gaa caa gag cac atg ctg aag gat tac ctg gtg gtg gcc 401
Trp Arg Arg Glu Gln Glu His Met Leu Lys Asp Tyr Leu Val Val Ala
        90                  95                  100

cag gag gct ctg agt gca caa aag gag atc tac cag gtg aag cag cag 449
Gln Glu Ala Leu Ser Ala Gln Lys Glu Ile Tyr Gln Val Lys Gln Gln
    105                 110                 115

cgc ctg gag ctg gca cag cag gag tat cag cag ttg cat gcc gtc tgg 497
Arg Leu Glu Leu Ala Gln Gln Glu Tyr Gln Gln Leu His Ala Val Trp
120                 125                 130                 135

gag cat aag ctg ggc tcc cag gtt agc ttg gtc tcg gga tcc tcc tcc 545
Glu His Lys Leu Gly Ser Gln Val Ser Leu Val Ser Gly Ser Ser Ser
                140                 145                 150

agc tca aag tat gac cct gag atc ctg aaa gct gaa att gcc acc gca 593
Ser Ser Lys Tyr Asp Pro Glu Ile Leu Lys Ala Glu Ile Ala Thr Ala
            155                 160                 165

aag tcc cgg gtg aac aaa ctg aag aga gag atg gtc cac cta cag cac 641
Lys Ser Arg Val Asn Lys Leu Lys Arg Glu Met Val His Leu Gln His
        170                 175                 180

gag cta cag ttc aag gag cgt ggc ttt cag acc ctg aag aaa att gat 689
Glu Leu Gln Phe Lys Glu Arg Gly Phe Gln Thr Leu Lys Lys Ile Asp
    185                 190                 195

gag agg atg tcc gat gct cag ggt ggc tac aaa ctg gac gaa gcc cag 737
Glu Arg Met Ser Asp Ala Gln Gly Gly Tyr Lys Leu Asp Glu Ala Gln
200                 205                 210                 215

gct gtg ctg agg gaa acc aaa gcc atc aag aaa gcc atc acc tgc gga 785
Ala Val Leu Arg Glu Thr Lys Ala Ile Lys Lys Ala Ile Thr Cys Gly
                220                 225                 230

gaa aag gag aaa caa gac ctg atc aag agt ctc gcc atg ctg aag gac 833
Glu Lys Glu Lys Gln Asp Leu Ile Lys Ser Leu Ala Met Leu Lys Asp
            235                 240                 245

ggc ttc cgg acg gac aga ggg tcg cac tcc gac ctg tgg tcc agc agc 881
Gly Phe Arg Thr Asp Arg Gly Ser His Ser Asp Leu Trp Ser Ser Ser
        250                 255                 260

agc tct ctg gag agt tcg agc ttc cca gtg cca aag cag ttc ctg gat 929
Ser Ser Leu Glu Ser Ser Ser Phe Pro Val Pro Lys Gln Phe Leu Asp
    265                 270                 275

gtg agc tcg cag aca gac atc tct ggg agt ttc agc acc agc agc agc 977
Val Ser Ser Gln Thr Asp Ile Ser Gly Ser Phe Ser Thr Ser Ser Ser
280                 285                 290                 295

aac cag ttg gcc gag aag gtc cga ttg cgc ctt cgg tat gaa gag gca 1025
Asn Gln Leu Ala Glu Lys Val Arg Leu Arg Leu Arg Tyr Glu Glu Ala

300                 305                 310

aag aga agg atc gcc aac ctc aag atc cag ctg gcc aaa ctt gac agt     1073
Lys Arg Arg Ile Ala Asn Leu Lys Ile Gln Leu Ala Lys Leu Asp Ser
            315                 320                 325

gag gcc tgg cct gga gtg cta gac tca gag agg gac cga ctg atc ctc     1121
Glu Ala Trp Pro Gly Val Leu Asp Ser Glu Arg Asp Arg Leu Ile Leu
        330                 335                 340

atc aat gag aag gag gag ctt ctg aag gag atg cgc ttc atc agc ccc     1169
Ile Asn Glu Lys Glu Glu Leu Leu Lys Glu Met Arg Phe Ile Ser Pro
    345                 350                 355

cgg aaa tgg acg cag ggt gaa gtg gag cag cta gag atg gcc cgg agg     1217
Arg Lys Trp Thr Gln Gly Glu Val Glu Gln Leu Glu Met Ala Arg Arg
360                 365                 370                 375

aga ctg gag aag gac ttg cag gca gcc cgg gac acc cag agc aag gcg     1265
Arg Leu Glu Lys Asp Leu Gln Ala Ala Arg Asp Thr Gln Ser Lys Ala
                380                 385                 390

ctg act gag agg ttg aag tta aac agc aag agg aac cag ctg gtg cga     1313
Leu Thr Glu Arg Leu Lys Leu Asn Ser Lys Arg Asn Gln Leu Val Arg
            395                 400                 405

gaa ctg gag gaa gcc acg cgg cag gtg gcc gcc ctg cac tct cag ttg     1361
Glu Leu Glu Glu Ala Thr Arg Gln Val Ala Ala Leu His Ser Gln Leu
        410                 415                 420

aaa agc ctc tcc agc agt atg caa tca ctg tct tca ggc agt agc cct     1409
Lys Ser Leu Ser Ser Ser Met Gln Ser Leu Ser Ser Gly Ser Ser Pro
    425                 430                 435

ggg tca ctt acg tcc agc cgg ggc tcc ctg gct gcc tct agc ctg gac     1457
Gly Ser Leu Thr Ser Ser Arg Gly Ser Leu Ala Ala Ser Ser Leu Asp
440                 445                 450                 455

tcc tcc acc tca gcc agc ttc act gac ctc tat tac gac ccg ttc gag     1505
Ser Ser Thr Ser Ala Ser Phe Thr Asp Leu Tyr Tyr Asp Pro Phe Glu
                460                 465                 470

cag ctg gac tca gag ctc ccg agc aaa gta gaa ctg ctg ttc ctg gag     1553
Gln Leu Asp Ser Glu Leu Pro Ser Lys Val Glu Leu Leu Phe Leu Glu
            475                 480                 485

ggt gcc aca ggc ttc cgg ccc tca ggc tgc atc acc acc atc cac gag     1601
Gly Ala Thr Gly Phe Arg Pro Ser Gly Cys Ile Thr Thr Ile His Glu
        490                 495                 500

gat gag gtg gcc aag act cag aag gca gag gga ggc agc cgc ctg cag     1649
Asp Glu Val Ala Lys Thr Gln Lys Ala Glu Gly Gly Ser Arg Leu Gln
    505                 510                 515

gcc ctg cgc tcc cta tcc ggc act cca aga tcc atg acc tcc ctg tct     1697
Ala Leu Arg Ser Leu Ser Gly Thr Pro Arg Ser Met Thr Ser Leu Ser
520                 525                 530                 535

cca cgc tca tct ctc tcc tcc ccg tcc cct ccc tgt tcc cct ctc atc 1745
Pro Arg Ser Ser Leu Ser Ser Pro Ser Pro Pro Cys Ser Pro Leu Ile
                540                 545                 550

gct gac ccc ctc ctg gct gga gat gcc ttt ctg acc cct ctg gag ttt 1793
Ala Asp Pro Leu Leu Ala Gly Asp Ala Phe Leu Thr Pro Leu Glu Phe
            555                 560                 565

gaa gac aca gag ctg agc ccc act ctt tgt gag ctg agc ctc ggc acc 1841
Glu Asp Thr Glu Leu Ser Pro Thr Leu Cys Glu Leu Ser Leu Gly Thr
        570                 575                 580

gat ggc gcc cgg gaa aga ttc cgg ctg gag gaa cca gga ccc gag ggc 1889
Asp Gly Ala Arg Glu Arg Phe Arg Leu Glu Glu Pro Gly Pro Glu Gly
    585                 590                 595

aaa cca ctg ggc cag gct gca agt gtg gcc cca ggg tgt ggc ctc aaa 1937
Lys Pro Leu Gly Gln Ala Ala Ser Val Ala Pro Gly Cys Gly Leu Lys
600                 605                 610                 615

gtg gcc tgt gtg tcc gct gca gtg tct gat gag tca gtg gcc ggg gac 1985
Val Ala Cys Val Ser Ala Ala Val Ser Asp Glu Ser Val Ala Gly Asp
                620                 625                 630

agc ggc gtg tat gag gct tcc atg cag aga cta ggt act tct gag gct 2033
Ser Gly Val Tyr Glu Ala Ser Met Gln Arg Leu Gly Thr Ser Glu Ala
            635                 640                 645

gct gca ttc gac agc gac gag tcg gaa gct gtg ggt gtc act cag gtt 2081
Ala Ala Phe Asp Ser Asp Glu Ser Glu Ala Val Gly Val Thr Gln Val
        650                 655                 660

cag att gcc ctg aaa tat gat gag aag agt aag cag ttt gca ata tta 2129
Gln Ile Ala Leu Lys Tyr Asp Glu Lys Ser Lys Gln Phe Ala Ile Leu
    665                 670                 675

gtc atc cag ctg agt aac ctc tct gcc ctg tta cta cag caa gat cag 2177
Val Ile Gln Leu Ser Asn Leu Ser Ala Leu Leu Leu Gln Gln Asp Gln
680                 685                 690                 695

aaa gtg aac atc cgt gta gcc atc ttg cct tgc tct gaa agc agc acc 2225
Lys Val Asn Ile Arg Val Ala Ile Leu Pro Cys Ser Glu Ser Ser Thr
                700                 705                 710

tgc ttg ttc aga acc cgg cct ctg gac tct gca gat agc ctt gtg ttc 2273
Cys Leu Phe Arg Thr Arg Pro Leu Asp Ser Ala Asp Ser Leu Val Phe
            715                 720                 725

aat gag gca ttc tgg gtg tcc atg tcc tac cca gcc ctt cac cag aag 2321
Asn Glu Ala Phe Trp Val Ser Met Ser Tyr Pro Ala Leu His Gln Lys
        730                 735                 740

acc tta cga gtt gat gtc tgc acc act gac agg agc cat aca gag gag 2369
Thr Leu Arg Val Asp Val Cys Thr Thr Asp Arg Ser His Thr Glu Glu
    745                 750                 755

tgc ctg gga ggt gcc cag atc agc ctg gct gag gtc tgc cgg tcc ggg 2417
Cys Leu Gly Gly Ala Gln Ile Ser Leu Ala Glu Val Cys Arg Ser Gly

760                 765                 770                 775

gag agg tca acc cgc tgg tac aac ctt ctg agc tac aaa tac ttg aag      2465
Glu Arg Ser Thr Arg Trp Tyr Asn Leu Leu Ser Tyr Lys Tyr Leu Lys
                780                 785                 790

aaa cag ggc agg gag ccc cag cca gca gaa gct cca ggg ccc gac cat      2513
Lys Gln Gly Arg Glu Pro Gln Pro Ala Glu Ala Pro Gly Pro Asp His
            795                 800                 805

gtg gat gcc gtg tct gcc ttg cta gaa cag aca gct gtg gaa cta gag      2561
Val Asp Ala Val Ser Ala Leu Leu Glu Gln Thr Ala Val Glu Leu Glu
        810                 815                 820

aag agg cag gag ggg aga agc agc tcc cag aca ctg gag ggc agc tgg      2609
Lys Arg Gln Glu Gly Arg Ser Ser Ser Gln Thr Leu Glu Gly Ser Trp
    825                 830                 835

acg tac gag gaa gag gcc agc gag aat gag gca gta gct gag gag gaa      2657
Thr Tyr Glu Glu Glu Ala Ser Glu Asn Glu Ala Val Ala Glu Glu Glu
840                 845                 850                 855

gag aga gag gaa gag gag gga gag gaa gat gtc ttc gct gaa aag gtc      2705
Glu Arg Glu Glu Glu Glu Gly Glu Glu Asp Val Phe Ala Glu Lys Val
                860                 865                 870

tcc ccg gag gca gag gag tgc cca gca tta aag gtg gac aga gag acc      2753
Ser Pro Glu Ala Glu Glu Cys Pro Ala Leu Lys Val Asp Arg Glu Thr
            875                 880                 885

aat act gac agc gtg acc cca tcc ccc aca gtg gtg cgc ccc aaa gac      2801
Asn Thr Asp Ser Val Thr Pro Ser Pro Thr Val Val Arg Pro Lys Asp
        890                 895                 900

cgg cgg gtg ggt gcc ccc tcg cca ggg cca ttt ctt cga ggg aat acc      2849
Arg Arg Val Gly Ala Pro Ser Pro Gly Pro Phe Leu Arg Gly Asn Thr
    905                 910                 915

atc atc cgc tcc aag acc ttc tcg cct gga ccc cag agc cag tat gtg      2897
Ile Ile Arg Ser Lys Thr Phe Ser Pro Gly Pro Gln Ser Gln Tyr Val
920                 925                 930                 935

tgt cgg ctg aat cga agc gac agc gac agc tcc acg ctg tcc aag aag      2945
Cys Arg Leu Asn Arg Ser Asp Ser Asp Ser Ser Thr Leu Ser Lys Lys
                940                 945                 950

cca ccc ttt gtt cga aac tcc ctg gag cgg cgc agc gtc agg atg aag      2993
Pro Pro Phe Val Arg Asn Ser Leu Glu Arg Arg Ser Val Arg Met Lys
            955                 960                 965

cgg cct tcg tcc gtc aag tcg ctg cgc aca gag cgc ctg atc cgc acc      3041
Arg Pro Ser Ser Val Lys Ser Leu Arg Thr Glu Arg Leu Ile Arg Thr
        970                 975                 980

tcg ctg gac cta gaa cta gac ctg cag gcc acg agg acc tgg cac agc      3089
Ser Leu Asp Leu Glu Leu Asp Leu Gln Ala Thr Arg Thr Trp His Ser
    985                 990                 995

cag ctg act cag gag atc tca gtg ctg aag gaa ctg aag gag cat 3134
Gln Leu Thr Gln Glu Ile Ser Val Leu Lys Glu Leu Lys Glu His
1000                1005                1010

ttg gag caa gcc aag aac cac ggg gag aag gag ctg cca caa tgg 3179
Leu Glu Gln Ala Lys Asn His Gly Glu Lys Glu Leu Pro Gln Trp
1015                1020                1025

ctg cgt gaa gat gag cgc ttc cgc ctc cta ctg agg atg ctg gag 3224
Leu Arg Glu Asp Glu Arg Phe Arg Leu Leu Leu Arg Met Leu Glu
1030                1035                1040

aag agg gtg gat cgc ggg gag cac aag agc gag ttg cag gcc gac 3269
Lys Arg Val Asp Arg Gly Glu His Lys Ser Glu Leu Gln Ala Asp
1045                1050                1055

aag atg atg agg gca gct gcc aag gac gtg cac agg ctc cga ggc 3314
Lys Met Met Arg Ala Ala Ala Lys Asp Val His Arg Leu Arg Gly
1060                1065                1070

cag agc tgt aag gag ccc ccg gaa gtg cag tct ttc agg gag aaa 3359
Gln Ser Cys Lys Glu Pro Pro Glu Val Gln Ser Phe Arg Glu Lys
1075                1080                1085

atg gca ttt ttc acc cgg cct cgg atg aac atc cca gct ctc tct 3404
Met Ala Phe Phe Thr Arg Pro Arg Met Asn Ile Pro Ala Leu Ser
1090                1095                1100

gcc gat gac gtc taa ccaccagaaa agtatttcct ttcttgcacg gaccatgctg 3459
Ala Asp Asp Val
1105

tgacggctga ctgtgactaa agttatttat gaggcattat gtgaaggtac cgagtcacag 3519

gtccccgagc gctctcgttg gtttgaagac aaacagattt ttttttttag ttcaggtttt 3579

cttgatatta tttaaaaaaa aaatgacaaa gcaaccataa caacaacaac aaaaacaaac 3639

aaacaaacaa acaaaaaaac cagcattaaa atgacaagat tgtatagttt gtatatttag 3699

gaatgtattt ttgggaaaga atttaaatga actaaagcag cgttaagcgg ctgctagtct 3759

taaaattgtc tagagttttc tttttgtaca gcaacaactt ttagaggttt ccctgcaata 3819

aaaaaaaaaa aaagaagtcg catttgaagg gcgataatcc tccgaggagg tcccgcaggt 3879

ggcacggcac agctaacctg tcctagttaa cattattttg tacaataata caaagaggag 3939

gatgtacaga aatcgtgggg ttgtcgcaga ctgtcctcgg atctttgtga gcttcgatgt 3999

gtcttcgtgt ggccacctga cctgaagcag gcagcgtgaa gctggctggg caaggccagc 4059

ccagacaggc ggcaggccac gcccatctca gatacccagt gacactgctg cgggagtgag 4119

gggagggccg cagagaactg catttggatc tctcgctatc aataaagtgg ccttttaaga 4179

aa 4181

<210>  6
<211>  1108
<212>  PRT
<213>  Rattus norvegicus

<400>  6

Met Pro Arg Pro Glu Leu Pro Leu Pro Glu Gly Trp Glu Glu Ala Arg
1               5                   10                  15

Asp Phe Asp Gly Lys Val Tyr Tyr Ile Asp His Arg Asn Arg Thr Thr
            20                  25                  30

Ser Trp Ile Asp Pro Arg Asp Arg Tyr Thr Lys Pro Leu Thr Phe Ala
        35                  40                  45

Asp Cys Ile Ser Asp Glu Leu Pro Leu Gly Trp Glu Glu Ala Tyr Asp
    50                  55                  60

Pro Gln Val Gly Asp Tyr Phe Ile Asp His Asn Thr Lys Thr Thr Gln
65                  70                  75                  80

Ile Glu Asp Pro Arg Val Gln Trp Arg Arg Glu Gln Glu His Met Leu
                85                  90                  95

Lys Asp Tyr Leu Val Val Ala Gln Glu Ala Leu Ser Ala Gln Lys Glu
            100                 105                 110

Ile Tyr Gln Val Lys Gln Gln Arg Leu Glu Leu Ala Gln Gln Glu Tyr
        115                 120                 125

Gln Gln Leu His Ala Val Trp Glu His Lys Leu Gly Ser Gln Val Ser
    130                 135                 140

Leu Val Ser Gly Ser Ser Ser Ser Ser Lys Tyr Asp Pro Glu Ile Leu
145                 150                 155                 160

Lys Ala Glu Ile Ala Thr Ala Lys Ser Arg Val Asn Lys Leu Lys Arg
                165                 170                 175

Glu Met Val His Leu Gln His Glu Leu Gln Phe Lys Glu Arg Gly Phe
            180                 185                 190

Gln Thr Leu Lys Lys Ile Asp Glu Arg Met Ser Asp Ala Gln Gly Gly
        195                 200                 205

Tyr Lys Leu Asp Glu Ala Gln Ala Val Leu Arg Glu Thr Lys Ala Ile
    210                 215                 220

Lys Lys Ala Ile Thr Cys Gly Glu Lys Glu Lys Gln Asp Leu Ile Lys
225                 230                 235                 240

Ser Leu Ala Met Leu Lys Asp Gly Phe Arg Thr Asp Arg Gly Ser His
                245                 250                 255

Ser Asp Leu Trp Ser Ser Ser Ser Ser Leu Glu Ser Ser Ser Phe Pro
            260                 265                 270

Val Pro Lys Gln Phe Leu Asp Val Ser Ser Gln Thr Asp Ile Ser Gly
        275                 280                 285

Ser Phe Ser Thr Ser Ser Ser Asn Gln Leu Ala Glu Lys Val Arg Leu
    290                 295                 300

Arg Leu Arg Tyr Glu Glu Ala Lys Arg Arg Ile Ala Asn Leu Lys Ile
305                 310                 315                 320

Gln Leu Ala Lys Leu Asp Ser Glu Ala Trp Pro Gly Val Leu Asp Ser
                325                 330                 335

Glu Arg Asp Arg Leu Ile Leu Ile Asn Glu Lys Glu Glu Leu Leu Lys
            340                 345                 350

Glu Met Arg Phe Ile Ser Pro Arg Lys Trp Thr Gln Gly Glu Val Glu
        355                 360                 365

Gln Leu Glu Met Ala Arg Arg Arg Leu Glu Lys Asp Leu Gln Ala Ala
    370                 375                 380

Arg Asp Thr Gln Ser Lys Ala Leu Thr Glu Arg Leu Lys Leu Asn Ser
385                 390                 395                 400

Lys Arg Asn Gln Leu Val Arg Glu Leu Glu Glu Ala Thr Arg Gln Val
                405                 410                 415

Ala Ala Leu His Ser Gln Leu Lys Ser Leu Ser Ser Ser Met Gln Ser
            420                 425                 430

Leu Ser Ser Gly Ser Ser Pro Gly Ser Leu Thr Ser Ser Arg Gly Ser
        435                 440                 445

Leu Ala Ala Ser Ser Leu Asp Ser Ser Thr Ser Ala Ser Phe Thr Asp
    450                 455                 460

Leu Tyr Tyr Asp Pro Phe Glu Gln Leu Asp Ser Glu Leu Pro Ser Lys
465                 470                 475                 480

Val Glu Leu Leu Phe Leu Glu Gly Ala Thr Gly Phe Arg Pro Ser Gly
                485                 490                 495

Cys Ile Thr Thr Ile His Glu Asp Glu Val Ala Lys Thr Gln Lys Ala
            500                 505                 510

Glu Gly Gly Ser Arg Leu Gln Ala Leu Arg Ser Leu Ser Gly Thr Pro
        515                 520                 525

Arg Ser Met Thr Ser Leu Ser Pro Arg Ser Ser Leu Ser Ser Pro Ser
    530                 535                 540

Pro Pro Cys Ser Pro Leu Ile Ala Asp Pro Leu Leu Ala Gly Asp Ala
545                 550                 555                 560

Phe Leu Thr Pro Leu Glu Phe Glu Asp Thr Glu Leu Ser Pro Thr Leu
                565                 570                 575

Cys Glu Leu Ser Leu Gly Thr Asp Gly Ala Arg Glu Arg Phe Arg Leu
            580                 585                 590

Glu Glu Pro Gly Pro Glu Gly Lys Pro Leu Gly Gln Ala Ala Ser Val
        595                 600                 605

Ala Pro Gly Cys Gly Leu Lys Val Ala Cys Val Ser Ala Ala Val Ser
    610                 615                 620

Asp Glu Ser Val Ala Gly Asp Ser Gly Val Tyr Glu Ala Ser Met Gln
625                 630                 635                 640

Arg Leu Gly Thr Ser Glu Ala Ala Ala Phe Asp Ser Asp Glu Ser Glu
                645                 650                 655

Ala Val Gly Val Thr Gln Val Gln Ile Ala Leu Lys Tyr Asp Glu Lys
            660                 665                 670

Ser Lys Gln Phe Ala Ile Leu Val Ile Gln Leu Ser Asn Leu Ser Ala
        675                 680                 685

Leu Leu Leu Gln Gln Asp Gln Lys Val Asn Ile Arg Val Ala Ile Leu
    690                 695                 700

Pro Cys Ser Glu Ser Ser Thr Cys Leu Phe Arg Thr Arg Pro Leu Asp
705                 710                 715                 720

Ser Ala Asp Ser Leu Val Phe Asn Glu Ala Phe Trp Val Ser Met Ser
                725                 730                 735

Tyr Pro Ala Leu His Gln Lys Thr Leu Arg Val Asp Val Cys Thr Thr
            740                 745                 750

Asp Arg Ser His Thr Glu Glu Cys Leu Gly Gly Ala Gln Ile Ser Leu
        755                 760                 765

Ala Glu Val Cys Arg Ser Gly Glu Arg Ser Thr Arg Trp Tyr Asn Leu
    770                 775                 780

Leu Ser Tyr Lys Tyr Leu Lys Lys Gln Gly Arg Glu Pro Gln Pro Ala
785                 790                 795                 800

Glu Ala Pro Gly Pro Asp His Val Asp Ala Val Ser Ala Leu Leu Glu
                805                 810                 815

Gln Thr Ala Val Glu Leu Glu Lys Arg Gln Glu Gly Arg Ser Ser Ser
            820                 825                 830

Gln Thr Leu Glu Gly Ser Trp Thr Tyr Glu Glu Glu Ala Ser Glu Asn
        835                 840                 845

Glu Ala Val Ala Glu Glu Glu Glu Arg Glu Glu Glu Glu Gly Glu Glu
    850                 855                 860

Asp Val Phe Ala Glu Lys Val Ser Pro Glu Ala Glu Glu Cys Pro Ala
865                 870                 875                 880

Leu Lys Val Asp Arg Glu Thr Asn Thr Asp Ser Val Thr Pro Ser Pro
                885                 890                 895

Thr Val Val Arg Pro Lys Asp Arg Arg Val Gly Ala Pro Ser Pro Gly
            900                 905                 910

Pro Phe Leu Arg Gly Asn Thr Ile Ile Arg Ser Lys Thr Phe Ser Pro
        915                 920                 925

Gly Pro Gln Ser Gln Tyr Val Cys Arg Leu Asn Arg Ser Asp Ser Asp
    930                 935                 940

Ser Ser Thr Leu Ser Lys Lys Pro Pro Phe Val Arg Asn Ser Leu Glu
945                 950                 955                 960

Arg Arg Ser Val Arg Met Lys Arg Pro Ser Ser Val Lys Ser Leu Arg
                965                 970                 975

Thr Glu Arg Leu Ile Arg Thr Ser Leu Asp Leu Glu Leu Asp Leu Gln
            980                 985                 990

Ala Thr Arg Thr Trp His Ser Gln Leu Thr Gln Glu Ile Ser Val Leu
        995                 1000                1005

Lys Glu Leu Lys Glu His Leu Glu Gln Ala Lys Asn His Gly Glu
    1010                1015                1020

Lys Glu Leu Pro Gln Trp Leu Arg Glu Asp Glu Arg Phe Arg Leu
    1025                1030                1035

Leu Leu Arg Met Leu Glu Lys Arg Val Asp Arg Gly Glu His Lys
    1040                1045                1050

Ser Glu Leu Gln Ala Asp Lys Met Met Arg Ala Ala Ala Lys Asp
    1055                1060                1065

Val His Arg Leu Arg Gly Gln Ser Cys Lys Glu Pro Pro Glu Val
    1070                1075                1080

Gln Ser Phe Arg Glu Lys Met Ala Phe Phe Thr Arg Pro Arg Met
    1085                1090                1095

Asn Ile Pro Ala Leu Ser Ala Asp Asp Val
    1100                1105

<210> 7
<211> 23

<212> DNA
<213> Artificial

<220>
<223> Primer for RT-PCR of human KIBRA

<400> 7
caccagaaga ccttaagagt cga 23


<210> 8
<211> 22
<212> DNA
<213> Artificial

<220>
<223> Primer for RT-PCR of human KIBRA

<400> 8
tcactatcac tccgattcag cc 22


<210> 9
<211> 21
<212> DNA
<213> Artificial

<220>
<223> Primer for RT-PCR of mouse and rat KIBRA

<400> 9
gaaggagctg aaggagcatt t 21


<210> 10
<211> 21
<212> DNA
<213> Artificial

<220>
<223> Primer for RT-PCR of mouse and rat KIBRA

<400> 10
cctgaaagac tgcacttctg g 21


<210> 11
<211> 3000
<212> DNA
<213> Homo sapiens

<400> 11
ttttttcaaa tcaaagactg agacttcagt gaagcaagct gcccaaggtc acacagctag 60

aaaaccgggt gggcttggga cccaaacagc ctgattctag agctcttgct tctcaaccaa 120

aaaactatga agcaaacatc aacagagatg aacatttgtc agtatcagtc atgttccaca 180

tcccaaccat ggggaagttc tgaggctgca gagtgattta aaaaagaaag aaagtctcca 240

atctgggtga cagagcgaga ctccgtctca aaaataaaaa gaaagaaaga aagaaagtca 300

gccaggcaag gtggctcatg tctgtaagcc cagcactttg ggaggctgag gtgggcggat 360

cacctgaggt caggagttcg agaccagcct ggccaatatg gtgaaactcc agctctactg 420

aaaaatacaa aaattagctg ggcgtggtgg cgcaggcctg taatcccagc tactctggaa 480

gctgaggcag gagaattgct tgaatctggg aggcagaggt tgtagtgagc cgagatcatg 540

ccactgcact ccagcctggg ggatgggggg agactccatc tcaaaaaaag aaaaaaagaa 600

agtataggtt aataactgag aacactgcac tagagtcaga cctagtctgt cttatgagct 660

ctgtatccca ggccaacaac aaagcatctt ggagcctcat ctgtaaaatg ggtataataa 720

atagactttc ctatgccaga gggttgttat gaagattgag atcatgtatt caaagaacct 780

agcaccaggt tggctttgct ttggggctac tgctctaatc aatagtgggt ttttatgtag 840

taaaggcttg acccaaacag aggaagtgta gagggctgtg ggaattcccc cataaaggaa 900

tggggaattc caacttttgg gtgttgaggg ccagtctgag gctggtgtcc ctggactctg 960

gtgcctggta tttatggaat gaaggagggc caagcactga gccccgaggc ctggcctcag 1020

ggaaggaatt aactatgaag aaggcatttg gaggcgaggc tcactctggt gggtcttttc 1080

tgtcaagagg agatgagacc acaaacatga ttccctctct ggctcagcaa caccctggtc 1140

catacagcac agggtgctgg ttcttctgga agaccgtggg gctcaggcaa gagaagacac 1200

ccaactgggc ttcattcgac cagtccagct tcctgccata tgccgagggc aaaaaagggt 1260

taaattccca ctcagtacac agatgtaaga ggagatgggg aagtgaacag ccagttgatc 1320

attcactcaa tcatgctagc ttgctttttg tatttaatgc tacattttaa atttatttat 1380

ttatttattt gagacagagt ctcgctttgt tgcccaggct ggagtgcagt ggtgcgatct 1440

tggctcactg caacctccac ctcccaggtt gaagtgattc tcatgcctca gcctcccgag 1500

tagctgggac tacgggcgtg caccaccacg cccagctaat ttattattat tattttttag 1560

tagagatggg gttcactatg ttggccaggc tggtctgaaa tcctgacctc aggtaatctg 1620

tctgcctcac cctcccaaag tgctgggatt acaggcgtga gccaccgcac ccactaattc 1680

tacattttta atttctcaag aaacattgtg tctctgattg ttttattctc ttgaacattt 1740

ttcaagatgg aattttgctt tgcttacttt cttctttttc atagccccac ttctcccaaa 1800

tccataacac acctacctca aagtatcaca ggaaagaaag cctgtgaagc tgctagccac 1860

agcctgtcac aagtaagtcc tcaatttagc taacaataac aatggcatta ctattacttt 1920

tacaaagtgt tttccctgac accctctgga agtcctccca ggaaagcgga tccacagcgc 1980

tcctaatttt agggaccctg aggggagcca gctacatttc caaatggtgg ggaagggtgg 2040

agggtgggcg aaacgtcgta gggagagttg aatagaagga gacagatggt gcctgtgttg 2100

ggggaaggga ccagagatcc tggaaaactt atgtttccca attcgcaaga atgtttccat 2160

ttaaccactt cttgcctgac tcctgattct acaccacaga aaagcaggga gtttttaaga 2220

tttggggcca cctgtgccga gagcctggct actccgggtg gagagagaca ggagcgtttt 2280

aagtacaggc agagcggttt ttggcccagc cccctaggtc tgggacgcag gtgaggggcg 2340

gtctctcctt cccctggcag gggcagcgta agggtggccg gcggctggga ctgtcccacg 2400

agggggcgcg tctcgggccc taggacccgc cctcgaggcg ctgcgcgcgg ggaggggacc 2460

cgggaaccca ggcgcccggc gctccgcctc ccgtttccgg ggcagcgcgg ttacctgcac 2520

cgcccgagcc gcacctggcg gaggcagaag tcacaaaccc ggcgagctca gagcggtgga 2580

gcggagagga gggacggcac ccggctgcag ggaggaggga ggcggcagcg gcagcggcgg 2640

cgtggagggc gcagcgcgcc gcgcggcgga ggagggcaga cggcggcggc ggcggcgctc 2700

ggctcgctct gcccggccgg ctgggcgcgc acccgggctc gcaccgcgcc gctgcggacg 2760

cacatggcag cgtgagaggc cggcggcggg aggagcgggc ggcgccgggt cggggctgca 2820

gggccgcatg gacagcggcg ccaccccggc cggcccctac tagggccccc catctgcggg 2880

cgccaccccc cggatcatgg tgcctcggcg gccgcccggg ctaagagcgg ccggctggag 2940

ccgctgagcc cccgctgcgg ccgggagctg catgggggag cgccggcagc gcttgggaag 3000

<210> 12
<211> 3000
<212> DNA
<213> Mus musculus

<400> 12
tcagtttgga ttcctgtggt tcagtcacct gcagaataac aaccaactca acctatcata 60

aggctcagca gcttttacct ttggcgtgct tgccctgggc ctctgaatgc ctagagatga 120

ttcacccggc attgcagggg ggcaggccac catctgtaga ttacatcacc ctgcttgcag 180

cttctgggag ataacctggg atcctaaggc ctaagcttta aggaaccaat tgctgttcca 240

aggcatggtc taaaggtata gaggaacaag ccactggaaa atgccaagcg gtgggcatct 300

ggcatacaac acattcatcc aggaagcttg gaaaataagg cacacacaca cacacacaca 360

cacacacaca ccctagttgg atgtgtcata agcagacaag cgtacttttc aggaaggaaa 420

agacatgggt tgtctgtgtt ttttagcaga aaatagttca gttgagccac actttataaa 480

agtgtgctgg tcatcagatc agccagggtg ctccacgtgg accccttggt gatagcttaa 540
tcccttattt ttctcatttc tttctttctt tttttttttt ttttttttaa ttttttgaga 600
cagggtttct ctgtgttccc ctggctgtcc tggaactcac tctgtagacc aggctggctt 660
cgaactcaga aatccacctg cctctgcctc ccaagtgctg ggattaaagg cgtgcgccat 720
gatgccctgc ttctcatttc attttgctct tcccaaccag gtggagggac tcttcattat 780
cacatttttc tcaagggaga ggcaggagga tgctgagctc aaggccaccc tggactgcat 840
agcaagaatc taaaaaaaca ttacaaaccc tcgatctaaa tacaatgaag cattaatggt 900
tgacccttca accattggtt tcaccggtgc tagggtgctt gtgtctcttc ttagctccat 960
ccctggccat cttgaagttc agtgtcctca tctccagaac gaggagaagt acagtaccct 1020
atccagggct ttgtcgtgaa gattagctga gatcatactt gaaaacgcaa gcctcgtatc 1080
agatcagcac gtgttggaag tgatggttcg ttcacacttg tgatctcaca cttgggaggc 1140
agaggtagga aggggagata accatagtat aaggccaggc agagcgaggt ctcccaggca 1200
aaaactcaaa acagattggc tggtttggtt cagtgaggat gcatgcctgg agtttgatcc 1260
taacagaacg ggggaaggag ggcactagaa gtacttataa ttgtgaaggc tttgcccatc 1320
acaggaagtc tggagggaga actccccagc agttgggtgg gtggtgagga cctccctgag 1380
atctgagatc cctggcttcg aagtataata ttgatgggat ggaggaggag ctacacccgt 1440
cccctatgtc ctcagggcat ttagatttga ggagcccacc ctaaaacgcc ttgtgtatca 1500
aacaggggat ccccacacag tacagggagt taactggtta ctccccacat taagggattg 1560
attcagacaa ggcgctggac ttcgttcttc cagtccagtt tcctgcctca ggcacagtta 1620
catttgcact gggggaaaca ccaaacctaa gagggaactt tttcccctta attcaatatt 1680
gctaatgtct caagacattt acttattctt gtttcagggt ttgccttttc ttttctcctt 1740
tcttttctcc ttccttcctt ccttccttcc ttccttcctt ccttccttcc ttccttcctt 1800
ccttccttcc tccctctctc tctctttctt tctttctttc tttcttttcc ttctctctct 1860
ttctctctct ctctctctct ctctctctct ctctctctct ctctctctcc ctctccccct 1920
ccctttcctt ccttccttat ttatttattt attccccccc cccgtgtttg ctagtttgac 1980
tcagcctgga acttttggat ttaagtaatc cttcttcagc ctcttgagta gccgagagac 2040
aggtgcactt cactatgact ggcaaaatat gtccttcctt ctccttcctt gccacccgct 2100
ggaagtcctc ttaggaagca gatccatagc attcctaatt ttagggaccc taaggaaagc 2160
cagctacatt tgcaaattac ggagaggagg gttgaaacaa cacggggaga actgaatagg 2220

aggaagacag atggtgtgag tgctgggggg gaagggacga gagatcctgg aaaacttatg 2280

ttcgccgatt tgcaagaatg ttttcactta accacttctt gcctgactcc tggttctcaa 2340

ttacagaaaa cagggtgtgt caagatttgg ggccacacgc gccaagagcc cggacactct 2400

ggcgtagaga gagcctagcc tcctggggca gaacggggtg gtcggcgatt gggactgtcc 2460

cacgaggggg cgcgtctcgg gctctaggac ccgccctcgc cgcgctgggc gcggggagga 2520

gacccgggaa ccctggagcc cggcgctccg cctcccgttt ccggggcagc gcagttacct 2580

gcaccgccgg agccacacct ggcggaggca gaagtctcgg ggcgcgcggc ggggctccgg 2640

cagaggctgc gggcggcggc ggcggcggcg gcggcgtgga gcgcagcgcg cagcggcagg 2700

ggcaggcagg cggcggctcg ggccgctctc ctggctggct ggccgcggac tcgggccggc 2760

tgggcgccgc ggcgggcgca catggcagcg tgagaggcgg gcggcgtggg gctggggccg 2820

cagggccgca tggacagagg cgccaccccg gccggccccc gttagggccc ccgatccgtg 2880

ggcgccactc cccgatcatg ctgcctcggc ggccgcctgg acccaaagcg gccagcccga 2940

gccgctgagc cccgtggcgg ctgcgagctg catgggggag cgcgggccag gctcgggaag 3000

<210> 13
<211> 3000
<212> DNA
<213> Rattus norvegicus

<400> 13
   ctctctgcct ccgtctctcc atcccctgaa cgtttacttt attgcctttt tttattgcgt 60
   gtctatgaac atgggatcgc cggtaccttc aggccagagg tgtcagatcc cctggagcaa 120
   gagatatagg cagttacgag ctgccagatg tgggggctgg gaagtggaat ccacgttcta 180
   ttagaaagtg gtacatgctc ttacctggtg agcatccccc caccaccaca gcaccatccc 240
   ccaccccgcc attcaatttc tcctcctcat tctcatgaaa tattgcccca tctcttaaga 300
   caggtgtgga aagtggcctt tctttgttgt tgcagtgcac actgacttta agaccatgga 360
   tggggctggg gatttagctc agtggtagag cgcttaccta ggaagcgcaa ggccctgggt 420
   tcggtcccca gctccggggg gaaaaaaaga accaaaaaaa aaaaaaaaaa aaaaagacca 480
   tggccacctg tggcgttacc tccagcaagc tacctaacct tgagtgcaaa tgcagacttg 540
   gtgtggttgg tagtctctgc tcgatcaacc ctgacgatat ccgcactctc attttgtaag 600
   ccagagagcc ctaagaggaa ggccgggagg ctgcttgcct tcagcttgga ttcctgtggt 660
   ccagtcacct gtagaataac aaccaattca acctatcaca aggctcagca ggttttacct 720
   ttggcatgct tgcccggggc ctctgaatgc ctggagatga ttcaccaggc attgcagggg 780
   ggcaggccac catctgtaga ttacatcagc ctgctttcgg ctttcgggag ataacctggg 840
   atcctaaggc ctaagcttta aggaatggat ttctcttcca aggcatagtc taaaggtata 900
   aaggaacaag acactggaaa atgccagggg taacacactc acccaaaaag cttggaaaat 960
   aaggtacaca cacacacaca cacacacaca cacacactaa ctggacgtgt cataagcagc 1020
   caaacatact tttcaggaag aaaaagacat gggttatttg tttgttttta gcagaaacta 1080
   gttcagttgg tgatggctta agcccttatt tttctcactt cgtttggctt ttcccaatca 1140
   gttggaggga ctcttacgtt actatattct tctcagaggg agaggcggga ggatgttgag 1200
   cttgaggcca ccctagcctg catagcaaga atctaaaaaa tattcggaac ccagatgtag 1260
   atacaatgaa ggattgatgg ttgcccaagg caaaaactca aaacaattga ggacactcac 1320
   ttggcaagtg tgacacctgg ggtttgatcc ctgacagcac aggaagggga tgtgaaggct 1380
   ttgacccatc agaggaagtc tagaggaaga gagaaccctc ccactcttgg gtggtgagga 1440
   gctccctagg atctgagatc cctggctttg aagctaatat tgatggactg gaggaagaac 1500
   tatactcacc cccgatgtcc ccagggcatt aagacttggg gagcccaccc taaaacgcct 1560
   tgtctagcaa aaggagctga agcccaaaca ggggattccc acacagtaca gggagttgac 1620
   tggttactcc ccaaatgaag agattctgac aaggcactgg acttcattct tctagtccag 1680
   cttcctgcct caggcatagt taaatttgca ctggggaaac accagaccta agagtgaaca 1740
   ggcagagggc aaagccagag gattcttggc tggatgacac catcttcttt tcccccttga 1800
   ttcagtattg ttagtgtctc gaggcattta cttacattct tgttttttgg tttgcctttt 1860
   ctttttcttc tttttccttt tctttctttc ttcctttttt cttttctttc tttctttcct 1920
   tctttctttc tttctttcct tcttccttac tattaattta tttatcccct gtgtttgcta 1980
   ttttgacgtg gcctggaact tttagattta agcaatcttt cttcctcagc ctcttgagta 2040
   gctgataggc aggtgcactt cactatgact ggcaaaatat gtccttcctt ctcctccctt 2100
   gccacccgct ggaagtcctc ttagggaagc agatccattg cattcctaat tttagggacc 2160
   ccaaggaaag ccagccacat ttgcaaatta cggaggggag ggttgaaaca acacggggag 2220
   aattgaatag gagggagaca gatggtgtga gtgctggggg aagggacgag agatcctgga 2280
   aaacttatgt tcgccgattt gcaagaatgt tttcacttaa ccacttcttg cctgactcct 2340
   gagtctatct cacagaaaac agggtgtgtc aagatttggg gccacatgca ccaagagcct 2400
   ggacattcgg tgtagagaga gtctagcctc ctggggcaga gcaggggtcg tcggcgattg 2460
   ggactgtccc acgagggggc gcgtctcggg ctctaggacc cgccctcgcc gcgctgggcg 2520
   cggggaggag acccgggaac cccggagccc ggcgctccgc ctcccgtttc cggggcagcg 2580
   cagttacctg caccgccgga gccacacctg gcggaggcag aagtctcggg gcgcgcaacg 2640
   gggcgccggc agtggctgcg agaggcagcg gcggcgtgga gcgcagcgcg gggcggctgg 2700
   ggcaggcagg cggcggctcg ggccgctctc ccggctggct ggccgcgtac tcgggccggc 2760
   tgggcgccgc ggcgggcgca catggcagcg tgagaggcgg gcggcgcggg gctggggccg 2820
   cagggccgca tggacagagg cgccactccg gccggccccc gttagggccc ccgatccgcg 2880
   ggcgccaccc cccgatcatg ctgcctcggc ggccgcctgg acccaaagcg gccggcctga 2940
   gccgctgagc cccgtggcgg ccgcgagctg catgggggag cgcgggccag gctcgggaag 3000